(19) Europäisches Patentamt
European Patent Office
Office européen des brevets

(11) Veröffentlichungsnummer : **0 540 469 A1**

# (12) EUROPÄISCHE PATENTANMELDUNG

(21) Anmeldenummer : **92810815.8**

(22) Anmeldetag : **22.10.92**

(51) Int. Cl.$^5$ : **C07H 19/01,** A01N 43/90

(30) Priorität : **31.10.91 CH 3190/91**

(43) Veröffentlichungstag der Anmeldung : **05.05.93 Patentblatt 93/18**

(84) Benannte Vertragsstaaten : **AT BE CH DE DK ES FR GB GR IE IT LI LU MC NL PT SE**

(71) Anmelder : **Gesellschaft für Biotechnologische Forschung mbH (GBF)**
**Mascheroder Weg 1**
**W-3300 Braunschweig-Stöckheim (DE)**

(71) Anmelder : **CIBA-GEIGY AG**
**Klybeckstrasse 141**
**CH-4002 Basel (CH)**

(72) Erfinder : **Sutter, Marius, Dr.**
**Margarethenstrasse 75**
**CH-4102 Binningen (CH)**
Erfinder : **O'Sullivan, Anthony, Dr.**
**Habsburgerstrasse 38**
**CH-4055 Basel (CH)**
Erfinder : **Höfle, Gerhard, Prof. Dr.**
**Alter Weg 12a**
**W-3300 Braunschweig (DE)**
Erfinder : **Böhlendorf, Bettina, Dr.**
**Zimmerstrasse 2**
**W-3300 Braunschweig (DE)**
Erfinder : **Kiffe, Michael**
**Fallsteinstrasse 13**
**W-3300 Braunschweig (DE)**

(54) **Sopharen c Markolid-Derivate und ihre Verwendung als Mikrobizide.**

(57) Verbindung der Formel I

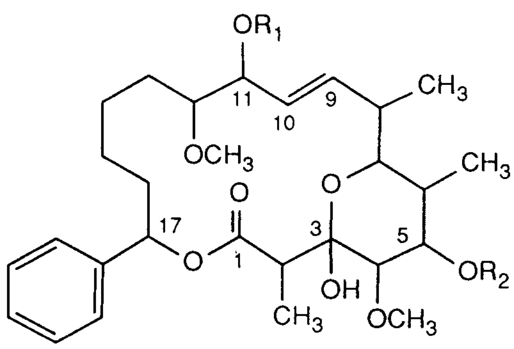

(I)

worin $R_1$ a) einen Kohlenwasserstoffrest mit maximal 15 C-Atomen darstellt, der gesättigt, ungesättigt, substituiert und/oder durch Heteroatome O, S, N unterbrochen sein kann, wobei die Bildung von (Thio)Acetalen oder Aminalen möglich ist ; b) Wasserstoff ist, falls $R_2$ ungleich Wasserstoff ist ; c) einen Acylrest $-COR_3$ darstellt, der einen gegebenenfalls substituierten Kohlenwasserstoff bedeutet (mit Einschluss eines Aminosäurerestes) und worin $R_2$ Wasserstoff, eine Silylgruppe oder einen Acylrest darstellt, besitzen mikrobizide Eigenschaften gegen pflanzenpathogene Pilze. Sie lassen sich als formulierte Schädlingsbekämpfungsmittel zum Schutz von Pflanzen gegen Krankheitsbefall einsetzen.

EP 0 540 469 A1

Die vorliegende Erfindung betrifft eine macrocyclische Verbindung der Formel I, ein Verfahren zu ihrer Herstellung und ihre Verwendung zur Bekämpfung von Pflanzenkrankheiten sowie pflanzenmikrobizide Mittel, die diese Verbindung als Wirkstoff enthalten.

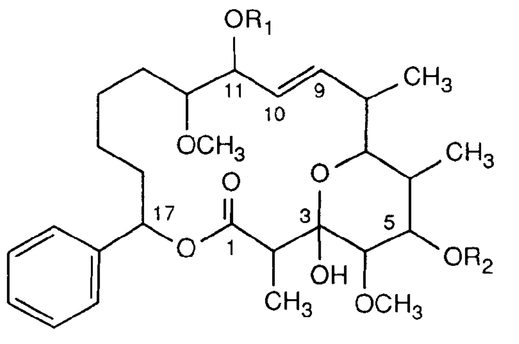

(I)

In der Formel I besitzt die Seitenkette $R_1$ maximal 15 Kohlenstoffatome und hat folgende Bedeutung:

- a) einen unsubstituierten oder substituierten Kohlenwasserstoffrest, ausgewählt aus $C_1$-$C_6$Alkyl, $C_3$-$C_6$Alkenyl, $C_3$-$C_6$Alkinyl, $C_3$-$C_6$Cycloalkyl, $C_3$-$C_6$Cycloalkyl, ($C_1$-$C_6$-)alkyl, Phenyl, Xylyl und Tolyl, wobei mögliche Substituenten aus Halogen, $CF_3$, Carboxy, Phenyl, mono- und disubstituiertem Phenyl, Hydroxy, $C_1$-$C_4$Alkoxy, $C_1$-$C_4$Alkylthio, Alkoxyalkoxy, Amino, Monoalkylamino, Dialkylamino ausgewählt sind; sowie einen gesättigten unsubstituierten oder substituierten heterocyclischen Fünf- oder Sechsring, wobei dieser Ring über ein zum Heteroatom O, S oder N $\alpha$-ständiges C-Atom mit dem Sauerstoffatom in 11-Position verbunden ist und derart ein cyclisches Acetal, Thioacetal bzw. Aminal bildet; mit der Massgabe, dass $R_1$ nicht Methyl ist;
- b) Wasserstoff, sofern der Substituent $R_2$ eine von Wasserstoff verschiedene Bedeutung hat;
- c) einen Acylrest -$COR_3$, worin $R_3$ Wasserstoff oder einen unsubstituierten oder durch Halogen, Phenyl, Phenoxy, $C_1$-$C_4$Alkoxy, $C_1$-$C_4$Alkylthio, Amino, Carboxy oder anderweitig nach Art einer Aminosäure substituierten Kohlenwasserstoffrest darstellt, der ausgewählt ist aus $C_1$-$C_6$Alkyl, $C_2$-$C_6$Alkenyl, $C_1$-$C_6$ Alkinyl, $C_3$-$C_6$Cycloalkyl; oder worin $R_3$ einen unsubstituierten oder substituierten Phenylrest oder einen kernsubstituierten Benzylrest darstellt, mit einem bis drei aus Methyl, Hydroxy, Methoxy, Nitro, Halogen und/oder Trifluormethyl ausgewählten Substituenten;

während $R_2$ die Bedeutung hat:

- Wasserstoff;
- eine Silylgruppe -SiR′R″R‴, worin R′, R″ und R‴ gleiche oder verschiedene Substituenten $C_1$-$C_6$Alkyl oder Phenyl darstellen;
- einen Acylrest -$COR_4$, in welchem $R_4$ Wasserstoff, Phenyl oder eine $C_1$-$C_6$Alkylgruppe darstellt, die unsubstituiert oder ein- oder mehrfach durch Halogen oder ein- bis zweifach durch $C_1$-$C_4$Alkoxy oder $C_3$-$C_6$Alkoxyalkoxy substituiert ist.

Die hier gegebenen allgemeinen Begriffe werden wie folgt erläutert.

Unter Halogen werden Fluor, Chlor, Brom oder Jod verstanden.

Unter $C_3$-$C_6$Cycloalkyl werden Cyclopropyl, Cyclobutyl, Cyclopentyl und Cyclohexyl verstanden.

Unter Alkyl werden je nach Kettenlänge Methyl, Ethyl, Propyl, Butyl, Amyl, Hexyl, sowie ihre Isomeren, z.B. Isopropyl, Isobutyl, sek. Butyl, tert. Butyl, Neopentyl usw. verstanden.

Ein durch Halogen substituierter Alkylrest steht für einen einfach bis perhalogenierten Alkylsubstituenten wie $CHCl_2$, $CH_2Cl$,, $CCl_3$, $CF_3$, $CH_2F$, $CH_2Br$, $CH_2CH_2Cl$, $CHF$-$CH_3$, $CHBr_2$ usw.

Alkenyl steht für einen aliphatischen Kohlenwasserstoffrest mit einer Doppelbindung, z.B. für Vinyl, Propen(1)-yl, Allyl, Buten(1)-yl, Buten(2)-yl, Hexen(2)-yl usw.

Alkinyl steht für einen aliphatischen Kohlenwasserstoffrest mit einer Dreifachbindung, z.B. für Ethinyl, Propin(1)-yl, Propargyl, Butin(1)-yl usw.

Als Substituenten für mono- oder disubstituiertes Phenyl kommen Methyl, Hydroxy, Methoxy, Nitro und/oder $CF_3$ in Frage.

Beispiele für Alkylreste, die durch Alkoxy oder Alkoxyalkoxy ein- oder mehrfach substituiert sind, seien -$CH_2CH_2OCH_3$, -$CH_2CH(CH_3)OCH_3$, -$CH_2CH_2CH_2OCH_3$, -$CH_2CH_2OCH_2OCH_3$, -$C(CH_3)_2$-$CH_2OCH_3$, -$CH_2CH_2$ $OCH_2CH_2OCH_3$ und andere verzweigte oder unverzweigte Reste.

In der 11-Position des Macrocyclus liegen Acetale, Thioacetale oder Aminale dann vor, wenn, wie angegeben, auf das Sauerstoffatom in dieser Position eine $C_1$-Brücke und danach wahlweise ein Sauerstoff-, Schwefel- bzw. Stickstoffatom folgen. Beispiele für offenkettige Derivate sind etwa -$CH_2OCH_3$, -$CH_2OC_2H_5$, -$CH_2OC_3H_7$(i),

$-CH(CH_3)OC_3H_7(i)$, $-CH_2OCH_2CH_2OCH_3$, $-CH(CH_3)OCH_3$, $-CH_2OC_4H_9(n)$, $-CH(CH_3)OCH_2CH_2-OCH_3$, $-CH(OCH_3)CH_2CH_2OCH_3$, $-CH_2-S-CH_3$, $-CH_2-S-CH_2CH_2OCH_3$, $-CH_2-N(C_2H_5)_2$, $-CH_2-N(CH_3)(C_2H_5)$. Als ringförmige Derivate seien α-Tetrahydropyran, α-Tetrahydrofuran, α-Tetrahydrothiopyran, α-Tetrahydrothiophen und α-Piperidin genannt, die nach Art einer Furanose oder Pyranose ganz oder teilweise durch -OH, $-CH_2OH$, $-CHOH-CH_2OH$, $NH_2$, $-CH_2NH_2$ oder durch entsprechende Acetylderivate substituiert sein können. Diese Aufzählung ist nicht limitierend.

Der Substituent $-COR_3$ kann den Rest einer (d)- oder (l)-Aminosäure darstellen, die mit der 11-Hydroxygruppe verestert ist. Beispiele von Aminosäuren sind Glycin, α- und β-Alanin, Valin, (Iso)Leucin, Phenylalanin, (Hydroxy)Prolin, Tyrosin, Serin, Threonin, Cystein, Methionin, Tryptophan, Asparaginsäure, Glytaminsäure (bzw. Pyrrolid-4-on-2-carbonsäure), Arginin, Lysin, Histidin, α- und γ-Aminobuttersäure u.a. Diese Aufzählung ist nicht limitierend.

Im engeren Sinne leitet sich die Verbindung der Formel I vorliegender Erfindung vom macrocyclischen Soraphen C her, wofür die folgende räumliche Struktur angenommen wird, und dessen Hemiacetalstruktur, abhängig vom pH-Wert und vom Lösungsmittel, zum Teil in der cyclischen Form und zum Teil in der seco-Form vorliegt:

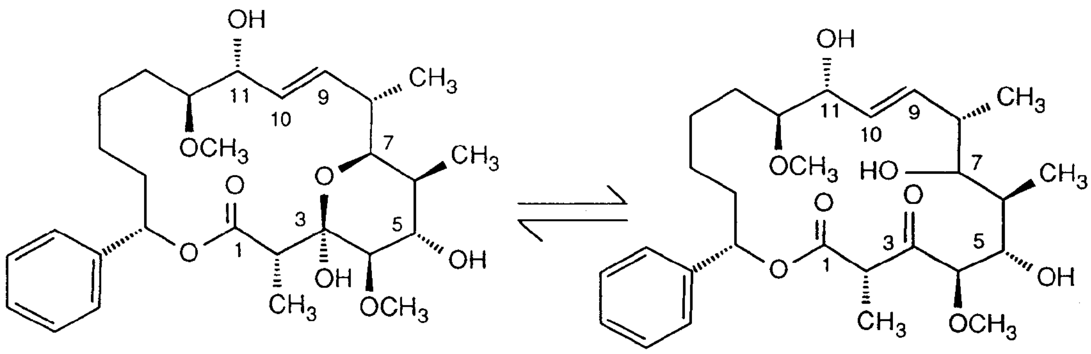

Soraphen C

cyclisches Hemiacetal seco-Form

Soraphen C ist aus der EP-A-0 358 606 als mikrobiologisch zugängliches Makrolid bekanntgeworden, das bei der Züchtung gewisser Sorangium cellulosum Stämme neben anderen Soraphenen gleicher Grundstruktur gebildet wird.

Die Derivate der hierin beschriebenen Formel I werden chemisch aus dem Soraphen C gewonnen. Es ist daher anzunehmen, dass die vorstehend angegebene räumliche Struktur des Soraphens C auch für die neuen Derivate gilt. Die vorliegende Erfindung bezieht sich auf alle isomeren Strukturen der Formel I und schliesst auch die mögliche seco-Form des 3,7-Hemiacetals mit ein. Desgleichen bezieht sich die Formel I auf alle möglichen Diastereomeren, die zusätzlich durch die Derivatisierung mit $R_1$ und/oder $R_2$ gebildet werden. Vereinfachend wird im folgenden die planare Schreibweise für die neuen Soraphen C-Derivate der Formel I beibehalten.

In Verbindungen der Formel I vorliegender Erfindung und in entsprechenden Zwischen-produkten ist die Reaktionsfähigkeit einer Hydroxylgruppe in 5-Stellung verschieden von der einer Hydroxylgruppe in 11-Stellung des Moleküls. Die Zugänglichkeit von z.B. in diesen Positionen unterschiedlich oder partiell substituierten Verbindungen ist daher gewährleistet.

Im Vergleich zur Wirkung des Grundkörpers Soraphen C entfalten die neuen Verbindungen der Formel I eine überraschend gesteigerte mikrobizide Wirkung.

Bevorzugt sind Verbindungen der Formel I, worin $R_1$ die genannte Bedeutung hat und $R_2$ Wasserstoff oder einen Acylrest-$COR_4$ bedeutet, in dem $R_4$ Wasserstoff oder eine $C_1$-$C_4$-Alkylgruppe bedeutet, die unsubstituiert ist oder durch $C_1$-$C_4$Alkoxy substituiert sein kann.

Diese Verbindungen sollen hier und im folgenden Untergruppe Ia genannt werden.

Unter diesen Verbindungen sind solche besonders bevorzugt, worin $R_1$ zusammen mit dem Sauerstoffatom in der 11-Position ein offenes Acetal der Formel

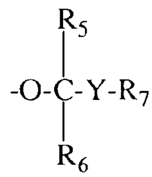

bedeutet, in dem Y Sauerstoff oder Schwefel, $R_5$ und $R_5$ unabhängig Wasserstoff oder $C_1$-$C_4$Alkyl und $R_7$ einen $C_1$-$C_4$Alkylrest darstellen, der unsubstituiert ist oder, im Falle $R_7$ = $C_2$-$C_4$Alkyl, β-ständig bis endständig (ω) mit 1-3 Substituenten, ausgewählt aus OH, Alkoxyalkoxy mit maximal 6 C-Atomen und $C_1$-$C_4$-Alkoxy, substituiert sein kann; oder worin $R_1$ ein ringförmiges Acetal der Formel

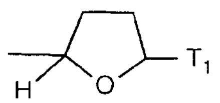 oder 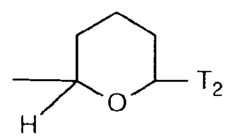

darstellt, das nach Art einer Furanose oder Pyranose ganz oder teilweise durch -OH oder -O-$COCH_3$ substituiert ist, und worin $T_1$ Wasserstoff, OH, -$CH_2OH$, -CHOH-$CH_2OH$ oder ihre Acetylderivate und $T_2$ Wasserstoff, OH, $CH_2OH$ oder deren Acetylderivate bedeuten;
oder worin $R_1$ ein Thioacetal der Formel

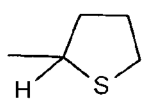 oder 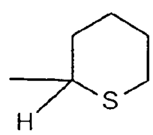

darstellt (Untergruppe Ib), insbesondere solche, bei denen $R_2$ Wasserstoff bedeutet (Untergruppe Ic).

Unter den Verbindungen der Untergruppe Ib sind solche Verbindungen bevorzugt, worin $R_1$ zusammen mit dem Sauerstoffatom in der 11-Position ein offenes Acetal der Formel

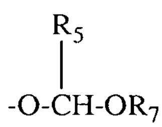

bedeutet, in dem $R_5$ Wasserstoff oder Methyl ist und $R_7$ einen $C_1$-$C_4$-Alkylrest darstellt, der unsubstituiert ist oder im Falle von $R_7$= $C_2$-$C_4$Alkyl β-ständig bis endständig durch ein bis drei $C_1$-$C_3$Alkoxygruppen substituiert sein kann (Untergruppe Id), insbesondere solche, bei denen $R_2$ Wasserstoff, Formyl, Acetyl oder Methoxyacetyl bedeutet (Untergruppe Ie).

Unter den Verbindungen der Formel Ia stellen auch jene eine wichtige Gruppe dar, bei denen $R_1$ einen $C_3$-$C_5$Cycloalkylrest oder einen unsubstituierten oder β-ständig bis endständig substituierten Kohlenwasserstoffrest bedeutet, ausgewählt aus $C_2$-$C_3$Alkyl, $C_3$-$C_4$Alkenyl und $C_3$-$C_4$Alkinyl, und dessen Substituenten ausgewählt sind aus OH, $C_1$-$C_4$Alkoxy und Alkoxyalkoxy mit maximal 6 C-Atomen. (Untergruppe If).

Innerhalb der Untergruppe If sind solche Verbindungen bevorzugt, bei denen $R_2$ Wasserstoff, Formyl, Acetyl oder Methoxyacetyl bedeutet (Untergruppe Ig).

Unter den Verbindungen der Formel I stellen weiterhin jene eine wichtige Untergruppe dar, bei denen $R_1$ den Carboxylrest -$COR_3$ einer Aminosäure darstellt (Untergruppe Ih), insbesondere jene, bei denen $R_2$ Wasserstoff, Formyl, Acetyl oder Methoxyacetyl bedeutet (Untergruppe Ii).

Eine wichtige Gruppe von Derivaten sind überdies jene Verbindungen der Formel I, bei denen $R_1$ Wasserstoff ist und $R_2$ eine Silylgruppe -SiR′R″R‴ bedeutet, worin R′, R″ und R‴ gleiche oder verschiedene Substituenten, ausgewählt aus $C_1$-$C_3$Alkyl und Phenyl darstellen. Diese Stoffgruppe Ii ist aufgrund ihres Charakters als Zwischenproduktegruppe für Derivatisierungen in 11-Position hervorzuheben.

Unter den besonders wichtigen Einzelverbindungen seien die folgenden genannt:

| | |
|---|---|
| -5-tert.Butyldimethylsilyl-Soraphen C | (Verb. Nr. 2) |
| -11-Methoxymethoxy-Soraphen C | (Nr. 68) |
| -11-Ethoxymethoxy-Soraphen C | (Nr. 82) |
| -11-Methylthiomethoxy-Soraphen C | (Nr. 73) |
| -11-Methoxyethoxymethoxy-Soraphen C | (Nr. 94) |
| -11-α-Tetrahydropyranyloxy-Soraphen C | (Nr. 108/109) |
| -11-Isopropoxy-Soraphen C | (Nr. 13) |
| -11-Allyloxy-Soraphen C | (Nr. 23) |
| -11-Methoxy-α-ethoxy-Soraphen C | (Nr. 104) |
| -11-Isopropoxymethoxy-Soraphen C | (Nr. 87) |
| -11-Isopropoxy-α-ethoxy-Soraphen C | (Nr. 224) |

Verbindungen der Formel I lassen sich in der 5-bzw. 11-Position, ausgehend vom Soraphen C nach Methoden derivatisieren, die gleichfalls Gegenstand vorliegender Erfindung sind.

Das Verfahren zur Herstellung von Verbindungen der Formel I ist gekennzeichnet durch Verätherung, Silylierung, (Thio)Acetalisierung, Aminalisierung, Acylierung, Silyletherspaltung und/oder Esterspaltung am "Soraphen C" in geeigneter Auswahl und Reihenfolge dieser Reaktionsschritte.

Durch übliche Acylierung einer OH-Gruppe mit der entsprechenden (Thio)Carbonsäure bzw. mit einem entsprechenden Acylhalogenid, Acylanhydrid oder Ester oder durch Reaktion der 5-OH-Gruppe mit dem entsprechend substituierten Silan-Derivat der Formel

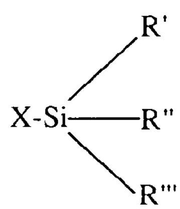

werden alle jene Derivate gewonnen, bei denen $R_1$ und/oder $R_2$ die Gruppe $-COR_3$ bzw. $-COR_4$ bedeuten, in der $R_3$ bzw. $R_4$ eine der für Formel I genannten Bedeutungen haben oder $R_2$ die Gruppe $-SiR'R''R'''$ darstellt, wobei der Begriff Acylhalogenid für Acylchlorid oder Acylbromid steht und wobei X eine Silylabgangsgruppe bedeutet. Zu den Silylabgangsgruppen X zählen beispielsweise Bromid, Chlorid, Trifluormethansulfonat. Diese Aufzählung stellt keine Limitierung dar. Der Fachmann kennt weitere typische Silylabgangsgruppen. Als geeignete Silylgruppen $-SiR'R''R'''$ kommen beispielsweise Trimethylsilyl, Diphenyl-tert.butylsilyl, bis(Isopropyl)-methylsilyl, Triphenylsilyl, Thexyldimethylsilyl usw. und insbesondere tert.Butyl-di-methylsilyl in Frage.

O-Acylierungen und O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0°C bis 80°C, bevorzugt bei 10°C bis 50°C ab. Vorzugsweise wird eine organische Base zugegeben. Genannt seien beispielsweise tertiäre Amine wie Triethylamin, Triethylendiamin, Triazol und bevorzugt Pyridin, Imidazol oder 1,8-Diazabicyclo[5.4.0]-undec-7-en (DBU).

Geeignete Lösungsmittel sind z.B.: Ether und etherartige Verbindungen wie Dialkylether (Diethylether, Diisopropylether, tert.-Butylmethylether, Dimethoxiethan, Dioxan, Tetrahydrofurn, Anisol, usw. ); halogenierte Kohlenwasserstoffe wie Chlorbenzol, Methylenchlorid, Ethylenchlorid, Chlororform, Tetrachlorkohlenstoff, Tetrachlorethylen, usw.; oder Sulfoxide wie Dimethylsulfoxid, wobei auch aromatische oder aliphatische Kohlenwasserstoffe, wie Benzol, Toluol, Xylole, Petrolether, Ligroin, Cyclohexan, usw. anwesend sein können. In manchen Fällen kann es von Vorteil sein, wenn die Reaktionen unter Schutzgas atmosphäre (z.B. Argon, Helium, Stickstoff, etc.) und/oder in absoluten Lösungsmitteln durchgeführt werden.

Setzt man zur Acylierung eine freie Carbonsäure als Reaktand ein, so wird diese Reaktion zweckmässigerweise in Gegenwart wasserabspaltender Reagenzien durchgeführt. Man arbeitet beispielsweise in Anwesenheit von Dicyclohexylcarbodiimid und Pyridin oder in Gegenwart von Azodicarbonsäuredialkylester und Triphenylphosphin.

Werden Säurehalogenide oder Säureanhydride zur Acylierung eingesetzt, so erweist sich der Zusatz eines Neutralisationsmittels als vorteilhaft. Tertiäre Amine wie beispielsweise Trialkylamine, Pyridin oder Pyridinbasen (wie 4-Dimethylaminopyridin) sind zweckmässige Reagenzien, die zum Teil auch als Lösungsmittel dienen können.

Die Herstellung von Verbindungen der Formel I, die in der 11-Position an das Sauerstoffatom einen Zuckerrest gebunden haben, wird nach einem der in der Zuckerchemie verwendeten Verknüpfungsmethoden, z.B. nach der Koenigs-Knorr-Synthese, dem Ag-Triflat-Prozess, dem Orthoester-Verfahren, der Phenylthio-Synthese, dem Trichloracetimidat-Verfahren oder der 2-Pyridylthio-Synthese durchgeführt.

A) Nach der Koenigs-Knorr-Synthese oder dem Silber-Triflat-Prozess lässt sich die 5-Hydroxy-Gruppe der Verbindung der Formel I ($R_2$=H) in Gegenwart eines Silbersalzes oder Quecksilber-Salzes als Kondensationsmittel mit dem einzuführenden Zuckerrest, worin sämtliche OH-Gruppen durch z.B. Acetylierung geschützt sind, mit Ausnahme der durch Chlor oder Brom substituierten 1-OH-Gruppen, im Temperaturbereich von -30°C bis +60°C, bevorzugt -5°C bis +30°C, unter Licht-Ausschluss gewinnen.

Der geschützte 1-Chlor- oder 1-Brom-Zucker wird in mindestens äquimolarer Menge zum Soraphen gegeben, vorzugsweise jedoch im 1,5- bis 3-fachen Ueberschuss.

Als Silbersalz lässt sich frisch gefälltes $Ag_2O$ verwenden, vorzugsweise jedoch $Ag_2CO_3$ oder $CF_3$-COOAg. Besonders bevorzugt ist Silber-Trifluormethansulfonat (=Ag-Triflat = $CF_3$-$SO_3Ag$), in dessen Gegenwart die Glykosidierung bereits bei Minustemperaturen schnell abläuft. Zum Aktivieren der 5-OH-Gruppe und zum Neutralisieren der gegebenenfalls entstehenden $CF_3$-$SO_3H$ bzw. $CF_3$-COOH setzt man zweckmässig ein tert.-Amin (Triethylamin, Diisopropylethylamin, Diazabicycloundecan u.a.) der Reaktionslösung zu.

Anstatt des Silbersalzes lässt sich auch Hg-Cyanid oder eine Kombination von HgO mit wahlweise Hg-Chlorid oder Hg-Bromid verwenden (Helferich-Synthese).

Nach einer weiteren Variante lässt sich die Reaktivität in der 1′-Stellung des glykosidisch zu verknüpfenden Zuckers, dessen weitere OH-Gruppen geschützt sein müssen, durch anfängliche Umwandlung in das 1′-Phenylthio-Derivat und anschliessende Reaktion mit DAST (= Diethylaminoschwefeltrifluorid) in absolut trockenem Dichlormethan (Molekularsieb) bei +5°C bis -30°C zum 1′-Fluorderivat erhöhen. Reaktiver als das entsprechende, bei der Koenigs-Knorr-Synthese eingesetzte 1′-Chlor- oder 1′-Brom-Derivat lässt sich das so gewonnene 1′-Fluor-Derivat des Zucker-Reaktanden mit der 5-Hydroxygruppe des Soraphens in Gegenwart von $SnCl_2$ und $AgClO_4$ in einem trockenen aprotischen Lösungsmittel wie Diethylether unter Schutzgas wie Argon bei +5°C bis -30°C verknüpfen. (J. Am. Soc. 1984, 106, 4189-4192).

B) Weiterhin kann man auch das geschützte, in 1′-Stellung zu aktivierende Monosaccharid mit 2,2′-Dithiopyridin in trockenem Dichlormethan bei -10°C bis +10°C und unter Schutzgas-Atmosphäre (z.B. Argon) in das 1′-S-(2-Pyridyl)-Monosaccharid überführen, das mit der freien 11-OH-Gruppe des Soraphens in Gegenwart von $Pb(ClO_4)_2$, $AgSO_3CF_3$ oder $AgClO_4$ als Kondensationsmittel bei Raumtemperatur in Tetrahydrofuran (THF) als Lösungsmittel leicht unter Bildung der glykosidischen Bindung reagiert. (J. Org. Chem. 1983, 48, 3489-3493).

Man kann auch das geschützte, in 1′-Stellung als Trichloracetimidat aktivierte Monosaccharid in trockenen aprotischen Lösungsmitteln mit Lewis-Säuren wie z.B. $(CH_3)_3SiOSO_2CF_3$ bei -50°C bis +50°C mit Soraphen C reagieren lassen.

C) Glykosidische Verknüpfungen lassen sich auch in Gegenwart von Lewis-Säuren erzielen, wie $AlCl_3$, $AlBr_3$, $SnCl_4$, $ZnCl_2$, $BF_3$ (sowie vor allem das Etherat), wozu insbesondere acetylierte Zucker sehr geeignet sind. (Chimia 21, 1967, S. 537-538).

D) Nach der sogenannten Orthoester-Methode lassen sich glykosidische Bindungen auch durch Reaktion von Soraphen mit dem zu verknüpfenden geschützten Zucker in Gegenwart des Orthoesters eines niederen Alkohols erzielen, dessen eine alkoholische Komponente der Zucker-Reaktand ist.

Das Verfahren zur Herstellung von Soraphen-11-Glykosiden der Formel I, ist im engeren Sinne gekennzeichnet durch Reaktion von Soraphen C

a) in Gegenwart eines Silbersalzes ode Quecksilber-Salzes als Kondensationsmittel mit dem einzuführenden Zuckerrest, dessen sämtliche OH-Gruppen geschützt sind, mit Ausnahme der in 1-Stellung durch Chlor oder Brom substituierten anomeren 1-OH-Gruppen, unter Licht-Ausschluss im Temperaturbereich von -30°C bis +60°C, bevorzugt -5°C bis +30°C; oder

b) in Gegenwart von $SnCl_2$ und $AgClO_4$ als Kondensationsmittel mit dem einzuführenden Zuckerrest, dessen sämtliche OH-Gruppen geschützt sind, mit Ausnahme der in 1-Stellung durch Fluor substituierten anomeren 1-OH-Gruppen unter Lichtausschluss bei +5°C bis -30°C;

und anschliessende milde Verseifung der Hydroxy-Schutzgruppen am Zuckerrest.

Die Schutzgruppen lassen sich in der Regel durch milde Verseifung mit z.B. $NH_3$/Methanol abspalten. Als Lösungsmittel kommen bei diesem Teilschritt insbesondere wasserfreie aprotische Vertreter wie Dichlormethan, Acetonitril, Benzol, Toluol, Nitromethan, Dioxan, THF, Ethylenglykoldimethylether in Frage; Diethylether ist besonders geeignet. Die Verätherung der 11-OH-Gruppe erfolgt als solche oder in der Alkali-Alkoholatform durch organische Halogenide oder Sulfate. Liegt die freie OH-Gruppe vor, arbeitet man vorzugsweise in Gegenwart von säurebindenden Mitteln wie Alkali(hydrogen)carbonat oder tert.Aminen wie Trialkylamin, 2,6-di-tert.Butylpyridin oder Pyridin. Als Halogenide kommen bevorzugt Bromide und Jodide wie Methyljodid, Propargylbromid, Allylbromid, Cyclopropylbromid, Cyclopentylbromid, vorteilhaft in Gegenwart katalytischer Mengen NaJ, in Frage. Niederalkylgruppen lassen sich vorteilhaft als Alkylsulfate einführen, z.B. Dimethylsulfat, Diethylsulfat und andere.

Als Lösungsmittel kommen beispielsweise Aceton, Dimethylformamid, Pyridin, 2,6-di-tert.Butylpyridin oder etherartige Verbindungen wie Tetrahydrofuran, Diethylether, 1,2-Dimethoxyethan in Frage. Die Reaktionstemperaturen liegen bei 0°C bis 100°C.

Gegebenenfalls ist auch die Zugabe eines Halogenfängers vorteilhaft. Hierzu eignen sich Silbersalze, z.B. $AgNO_3$, $AgOSO_2CF_3$ etc.

Sind im Molekül oder im Reaktanden störende funktionelle Gruppen wie OH, $NH_2$, -COOH, so können diese, wie bereits oben erwähnt, durch Acetylierung oder Einführung anderer Schutzgruppen einleitend maskiert und im Endprodukt wieder gespalten werden [T.W. Green "Protective Groups in Organic Synthesis", J. Wiley & Sons, 1981 (New York)].

Offene oder ringförmige Acetale (z.B. Methoxymethyl- oder Tetrahydropyranylether) lassen sich einerseits aus den entsprechenden 1-Halogen- 1-alkoxymethanen (z.B. Chloromethyl-methyläther, Bromomethyl-benzylether, Methoxy-ethoxy-methylchlorid etc.) in aprotischen Lösungsmitteln wie Ethern (z.B. Diethylether, Dimethoxyethan, Dioxan, Tetrahydrofuran usw.), halogenierten Kohlenwasserstoffen (Methylenchlorid, Chloroform, Chlorbenzol usw.), aliphatischen und aromatischen Kohlenwasserstoffen (wie Toluol, Xylol, Hexan, Petrolether usw.) Nitrilen(wie Acetonitril, Propionitril etc.), Estern (wie Ethylacetat, Propylacetat, Butylacetat etc), Ketonen (wie Aceton, Methylethylketon usw.), Dimethylsulfoxid, Dimethylformamid und anderen herstellen. Die Zugabe einer Base wie Natriumhydrid, Triethylamin, Diisopropylamin, Diisopropylethylamin, Pyridin, p-Dimethylaminopyridin, Kalium-tert.Butanolat usw. ist vorteilhaft. Die Reaktion wird bei Temperaturen von -50 bis 100°C durchgeführt, bevorzugt von -20 bis °25°C. Unter Umständen ist die Zugabe eines Halogenfängers wie z. B. Silbersalze (Silbernitrat, Silbertriflat usw.) etc. empfehlenswert. Andererseits lassen die Acetale sich aus den entsprechenden Enoläthern wie z.B. Dihydropyran, Methyl-vinyl-ether usw. in aprotischen Lösungsmitteln (wie beispielsweise den oben genannten) unter Säurekatalyse in die Acetale umwandeln. Als Säuren kommen z.B. wasserfreie Salzsäure, Schwefelsäure, p-Toluolsulfonsäure, Pyridinium-para-toluolsulfonat, Phosphoroxychlorid usw. oder aber auch mit sauren Gruppen modifizierte Polymere, wie z.B. Amberlit H-15, in Frage. Die Reaktion kann bei -50°C bis +100°C durchgeführt werden, bevorzugt bei -10°C bis +30°C.

Ein alternativer Syntheseweg ist die Umacetalisierung. Dabei werden Acetale wie Dimethoxy- oder Diethoxymethan etc. mit den entsprechenden Alkoholen unter Säurekatalyse umgesetzt. Die Reaktion kann dabei im Acetal selbst als Lösungsmittel oder aber unter Zugabe von wie oben genannten aprotischen Lösungsmitteln durchgeführt werden. Als saurer Katalysator kommen Phosphorpentoxid, Chlorwasserstoffsäure, p-Toluolsulfonsäure oder beispielsweise die oben genannten in Frage. Es kann vorteilhaft sein, den entstandenen Alkohol aus dem Reaktionsgemisch zu entfernen (Destillation, Molekularsieb o.ä.). Die Reaktionstemperatur beträgt -20° bis +100°C.

Für die übrigen weiter oben genannten Derivatisierungen der Soraphen-Struktur empfehlen sich im allgemeinen folgende Lösungsmittel oder Lösungsmittelgemische:

Aliphatische und aromatische Kohlenwasserstoffe wie Benzol, Toluol, Xylole, Petrolether, Hexan; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; Ester wie Ethylacetat, Propylacetat oder Butylacetat; Ketone wie Aceton, Diethylketon, Methylethylketon; Dimethylsulfoxid (DMSO); Dimethylformamid (DMF) und andere.

Die Aufzählung aller vorgenannten Methoden ist nicht limitierend. Gewünschtenfalls können die Endprodukte auf übliche Art und Weise gereinigt werden, z.B. durch Waschen, Digerieren, Extraktion, Umkristallisation, Chromatographie usw.

Die beschriebenen Herstellungsverfahren einschliesslich aller Teilschritte sind ein Bestandteil der vorliegenden Erfindung. Es wurde gefunden, dass Verbindungen der Formel I ein für praktische Bedürfnisse sehr günstiges biozides Spektrum gegen phytopathogene Mikroorganismen, insbesondere gegen Pilze, aufweisen. Sie besitzen sehr vorteilhafte kurative, systemische und insbesondere präventive Eigenschaften und werden zum Schutz von zahlreichen Kulturpflanzen eingesetzt. Mit den Wirkstoffen der Formel I können an Pflanzen oder Pflanzenteilen (Früchten, Blüten, Laubwerk, Stengel, Knollen, Wurzeln) von unterschiedlichen Nutzkulturen die auftretenden Schädlinge eingedämmt oder vernichtet werden, wobei auch später zuwachsende

Pflanzenteile vor phytopathogenen Mikroorganismen verschont bleiben.

Als Mirkobizide sind die Wirkstoffe der Formel I z.B. gegen die den folgenden Klassen angehörenden phytopathogenen Pilze wirksam: Fungi imperfecti (z.B. insbesondere Botrytis, ferner Pyricularia, Helminthosporium, Fusarium, Septoria, Cercospora und Alternaria); Basidiomyceten (z.B. Rhizoctonia, Hemileia, Puccinia). Darüber hinaus wirken sie gegen die Klasse der Ascomyceten (z.b. insbesondere Venturia und Erysiphe, ferner Podosphaera, Monilinia, Uncinula) und der Oomyceten (z.B. Phytophthora, Plasmopara). Die Verbindungen der Formel I können ferner als Beizmittel zur Behandlung von Saatgut (Früchte, Knollen, Körner) und Pflanzenstecklinge zum Schutz vor Pilzinfektionen sowie gegen im Erdboden auftretende phytopathogene Pilze eingesetzt werden.

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten Gebieten.

Eingeschlossen ist auch ein Verfahren zur Behandlung von Pflanzen, das sich durch Applikation der neuen Verbindungen der Formel I bzw. der entsprechenden neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfürchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln,

Tomaten, Kartoffeln, Paprika); Lorbeergewächse (Avocado, Cinnamonium, Kampfer) oder Pflanzen wie Tabak, Nüsse, Kaffee, Ananas, Zuckerrohr, Tee, Pfeffer, Weinreben, Hopfen, Bananen- und Naturkautschukgewächse sowie Zierpflanzen (z.B. Compositen). Diese Aufzählung stellt keine Limitierung dar.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden. Pflanzenöle wie Baumwollsamenöle, Rizinusöl, Flachsöl, Rapsöl, Olivenöl, Sonnenblumenöl, Maiskeimöl, Sesamöl etc. sind applikationsfördernde Zusätze.

Geeignete Träger und Zusätze können fest oder flüssig sein und entsprechen den in der Formulierungstechnik zweckdienlichen Stoffen, wie z.B. natürlichen oder regenerierten mineralischen Stoffe, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Verbindungen der Formel I können auch auf Vermehrungsgut wie Samenkörner Stecklinge, Knollen, Zweige, Wurzeln aufgebracht werden (Coating), indem man das Pflanzenmaterial entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulten, durch Verkapselungen in z.B. polymeren Stoffen in bekannter Weise verarbeitet. Die Anwendungsverfahren wie Versprühen, Vernebeln, Verstäuben, Verstreuen, Injizieren, Bestreichen oder Giessen werden gleich wie die Art der Mittel den angestrebten Zielen und den gegebenen Verhältnissen entsprechend gewählt. Günstige Aufwandmengen liegen im allgemeinen bei 5 g bis 500 g Aktivsubstanz (AS) pro Hektar, bevorzugt bei 50 g bis 200 g AS/ha. Bei Behandlung des Vermehrungsgutes betragen die Aufwandmengen 1 g bis 100 g AS/100 kg Pflanzenmaterial, z.B. Saatgut.

Die Formulierung d.h. die den Wirkstoff der Formel I und einen festen oder flüssigen Zusatzstoff enthaltenden Mittel werden in bekannter Weise hergestellt.

Als Lösungsmittel kommen in Frage: Aromatische und aliphatische Kohlenwasserstoffe, wie z.B. Xylolgemische, Cyclohexan oder Paraffine; dann Alkohole und Glykole sowie deren Ether und Ester, wie Ethanol,

Ethylenglykol, Ethylenglykolmonomethyl- oder ethylether, Essigsäureester, Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit, Zur verbesserung de physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien, z.B. Calcit oder Sand, in Frage. Darüber hinaus kann eine Vielzahl von vorgranulierten Materialien anorganischer Natur wie insbesondere Dolomit oder zerkleinerte Pflanzenrückstände verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Häufiger werden jedoch sog. synthetische Tenside verwendet, insbesondere Alkansulfonat, Fettalkoholsulfate, sulfonierte Benzimidazolderivete oder Alkylsulfonate.

Als nichtionische Tenside kommen Polyglykoletherderivate von aliphatischen doer cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Als weitere Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 95 % Wirkstoff der Formel I, 99,9 bis 5 % eines festen oder flüssigen Zusatzstoffes und 0 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken.

## Herstellungsbeispiele

### H-1. Herstellung von 5-tert. Butyldimethylsilyl-Soraphen C (Verb. Nr. 2)

15,0 g Soraphen C werden in 50 ml Dimethylformamid gehört und bei 0°C mit 17,8 g tert. Butyldimethylsilylchlorid und 8,05 g Imidazol versetzt. Nach 16 h Rühren bei Raumtemperatur wird auf Wasser gegossen, mit 3 x 50 ml Ethylacetat extrahiert, die organische Phase mit 1N Chlorwasserstoffsäure und gesättigter Kochsalzlösung gewaschen, über Natriumsulfat getrocknet und eingeengt. Chromatographie an Kieselgel mit Essigester/Hexan (1:9 bis 1:1) liefert 14,10 g (65 %) 5,11-Di-tertbutyldimethylsilyl-Soraphen C. Dieses wird 5 Stunden bei -20°C bis -10°C in einer Lösung von Fluorwasserstoff/Wasser/Acetonitril (2:3:95) gerührt. Das Reaktionsgemisch wird auf gesättigte $NaHCO_3$-Lösung gegossen und mit 4 x 20 ml Ethylacetat extrahiert. Die organische Phase wird mit gesättigter $NaHCO_3$-Lösung und gesättigter Kochsalzlösung (= Sole) gewaschen, über Natriumsulfat getrocknet und eingeengt. Chromatographie an Kieselgel mit Ethylacetat/Hexan (1:3 bis 1:1) liefert 2,92 g (21 %) Edukt und 8,74 (73 %) 5-tert.Butyldimethylsilyl-Soraphen C.

### H-2. Herstellung von 5-tert.Butyldimethylsilyl-11-ethoxy-Soraphen C (Verb.Nr. 11)

52,4 mg der Verbindung Nr. 2 werden in 2 ml Methylenchlorid gelöst und mit 180,9 mg 1,8-Bis-dimethylamino-naphthalin versetzt. Nach Zugabe von 54,2 ml Trifluormethansulfonsäureethylester wird während 18 Stunden bei Raumtemperatur gerührt. Danach werden 2 ml Triethylamin zugefügt, und es wird weitere 10 Minuten gerührt. Das Reaktionsgemisch wird in Ether aufgenommen, mit 1N Salzsäure, gesättigter $NaHCO_3$-

Lösung und gesättigter Kochsalzlösung gewaschen, über Natriumsulfat getrocknet und eingeengt. Chromatographie an Kieselgel mit Ethylacetat/Hexan (1:3) als Laufmittel liefert 31,4 mg der Titelverbindung.

H-3. Herstellung von 11-Ethoxy-Soraphen C (Verb. Nr. 10)

15 mg der Verbindung Nr. 11 werden während 2 Tagen in einer Lösung von Fluorwasserstoff/Wasser/Acetonitril (2:3:95) gerührt, anschliessend in Essigester aufgenommen, mit gesättigter Natriumhydrogencarbonat-Lösung und gesättigter Kochsalzlösung gewaschen, über Natriumsulfat getrocknet, filtriert und eingeengt. Chromatographie an Kieselgel mit Hexan/Essigsäureethylester (5:1) als Laufmittel liefert 7,6 mg von 11-Ethoxy-Soraphen C.

H-4. Herstellung von 5-tert.Butyldimethylsilyl-11-allyloxy-Soraphen C (Verb. Nr. 24)

52 mg der Verbindung Nr. 2 werden in 1 ml Tetrahydrofuran gelöst, mit 10 mg Natriumhydrid und 0,2 ml Allyljodid versetzt. Nach 2 Stunden Rühren bei Raumtemperatur wird das Reaktionsgemisch in 5 ml Ethylacetat aufgenommen, mit 1N Salzsäure und gesättigter Kochsalzlösung gewaschen, über Natriumsulfat getrocknet, filtriert und eingeengt. Chromatographie an Kieselgel mit Ethylacetat/Hexan (1:5 bis 1:1) ergibt 32,2 mg der Titelverbindung.

H-5. Herstellung von 11-Allyloxy-Soraphen C (Verb. Nr. 23)

32,2 mg der Verbindung Nr. 24 werden während 2 Tagen bei Raumtemperatur in 2 ml einer Lösung aus 7,8 g 70%iger Fluorwasserstoffsäure (in Pyridin gelöst) in 18,8 ml Pyridin und 60 ml Tetrahydrofuran gerührt. Das Reaktionsgemisch wird in Ethylacetat aufgenommen, mit 1N Salzsäure, gesättigter Natriumchloridlösung gewaschen, über Natriumsulfat getrocknet, filtriert und eingeengt. Chromatographie an Kieselgel mit Ethylacetat/Hexan (2:3 bis 2:1) als Laufmittel ergibt 11,6 mg der Titelverbindung.

H-6. Herstellung von 5-tert.Butyldimethylsilyl-11-benzyl-Soraphen C (Verb. Nr. 44)

534 µl 2,6-Di-tert.butyl-pyridin und 207 mg Silbertrifluormethansulfonat werden am Hochvakuum getrocknet und unter Argon sukzessive mit 50 mg der Verbindung Nr. 2 und 96 µl Benzylbromid versetzt. Nach 2 Minuten wird Ethylacetat zugegeben und die Suspension wird filtriert. Das Filtrat wird mit 1N Chlorwasserstoffsäure dann gesättigter Natriumhydrogencarbonatlösung und dann mit Sole gewaschen, über Natriumsulfat getrocknet, filtriert und eingeengt. Chromatographie an Kieselgel mit Ethylacetat/Hexan (1:10) als Laufmittel ergibt 30,9 mg der Titelverbindung.

H-7. Herstellung von 5-tert.Butyldimethylsilyl-11-methoxymethoxy-Soraphen C (Verb. Nr. 69)

60,8 mg der Verbindung Nr. 2 werden in 2 ml Methylenchlorid gelöst und mit 0,3 ml Diisopropylethylamin sowie 0,2 ml Brommethyl-methylether versetzt. Nach 16 Stunden Rührenlassen bei Raumtemperatur wird das Gemisch in Ethylacetat aufgenommen, mit 1N Chlorwasserstoffsäure, gesättigter Natriumhydrogencarbonatlösung und Sole gewaschen, über Natriumsulfat getrocknet, filtriert und eingeengt. Chromatographie an Kieselgel mit Ethylacetat/Hexan (1:3 bis 1:1) als Laufmittel liefert 39 mg der Titelverbindung.

H-8. Herstellung von 11-Methoxy-methoxy-Soraphen C (Verb. Nr. 68)

30,1 mg der Verbindung Nr. 69 werden während 4 Tagen bei Raumtemperatur in 2 ml einer Lösung, bestehend aus 7,8 g 70%iger Fluorwasserstoffsäure (in Pyridin gelöst) in einem Gemisch aus 18,8 ml Pyridin und 60 ml Tetrahydrofuran, gerührt. Danach wird das Reaktionsgemisch in Ethylacetat aufgenommen, mit 1N Chlorwasserstoffsäure, und dann mit gesättigter Natriumhydrogencarbonatlösung und Sole gewaschen, über Natriumsulfat getrocknet, filtriert und eingeengt. Chromatographie an Kieselgel mit Ethylacetat/Hexan (1:2 bis 1:1) ergibt 19,9 mg der Titelverbindung.

H-9. Herstellung von 5-tert.Butyldimethylsilyl-11-methylthiomethoxy-Soraphen C (Verb. Nr. 74)

162 mg der Verbindung Nr. 2 werden während 36 Stunden in einer Lösung von 1 ml Dimethylsulfoxid und 1 ml Essigsäureanhydrid bei Raumtemperatur eingeengt. Das Gemisch wird am Hochvakuum zur Trockene eingeengt, in Ethylacetat aufgenommen, mit gesättigter Natriumhydrogencarbonatlösung und Sole gewaschen, über Natriumsulfat getrocknet, filtriert und eingeengt. Chromatographie an Kieselgel mit Ethylacetat/Hexan (1:4 bis 1:2) als Laufmittel liefert 61,1 mg der Titelverbindung.

H-10. Herstellung von 5-tert.Butyldimethylsilyl-11-(β)-tetraacetylglukosidyl-Soraphen C (Verb. Nr. 134)

51,1 mg der Verbindung Nr. 2 wird mit 370 mg 1-Thiopyridylacetylglucose in 9 ml Toluol gelöst. Es wird 5 g Molekularsieb (4 Å) und 140 mg Silbertrifluormethansulfonat beigegeben. Nach 16 Stunden Rühren bei Raumtemperatur wird das Reaktionsgemisch auf Kieselgel gegeben und mit Ethylacetat/Hexan (1:1) chromatographiert. Dies ergibt 22 mg der Verb. Nr. 134.

H-11. Herstellung von Soraphen C-11-maleinat (Verb. Nr. 181)

100 mg (0.197 mmol) Soraphen C in 3 ml Dichlormethan wird mit 149 mg (1.519 mmol) Maleinsäureanhydrid und 171.5 mg (1.52 mmol) 2,4,6-Collidin versetzt und anschliessend bei 40°C während 3 h gerührt. Zur Aufarbeitung wird das Reakrionsgemisch mit 1N Chlorwasserstoffsäure hydrolysiert und mit Ethylacetat extrahiert. Die vereinigten organischen Phasen werden anschliessend nochmals mit 1N Chlorwasserstoffsäure sowie mit gesättigter Natriumchlorid-Lösung gewaschen und über Natriumsulfat getrocknet. Das Rohprodukt wird über PSC bzw. Säulenchromatographie gereinigt. Das erhaltene Soraphen C-5,11-dimaleinat wird in 1 ml Methanol gelöst und anschliessend mit 1 ml ammoniakalischer, wasserfreier Methanollösung (2 mmol $NH_3$/ml) versetzt. Der Reaktionsfortschritt wird mit Hilfe der Dünnschichtchromatographie beobachtet und nach ca. 4-8 h abgebrochen, bevor sich nennenswerte Mengen Soraphen C gebildet haben. Das Reaktionsgemisch wird mit 1N Chlorwasserstoffsäure versetzt und mit Ethylacetat extrahiert. Die vereinigten organischen Phasen werden nochmals mit 1N Chlorwasserstoffsäure sowie anschliessend mit gesättigter Natriumchlorid-Lösung extrahiert. Das Rohgemisch wird durch präparative Schichtchromatographie (Laufmittel: Dichlormethan/Methanol 90:10,1 % Essigsäure, v/v), anschliessend über Sephadex LH-20 (Säulenhöhe: 30 cm, Säulendurchmesser: 3 cm; Laufmittel: Methanol) gereinigt. Man erhält 32 mg der Verbindung Nr. 181.

H-12. Herstellung von 11-O-Tetrahydropyranyl-soraphen C (Verb. Nr. 108/109)

935 mg (1.846 mmol) Soraphen C werden in 10 ml Ethylacetat gelöst und mit 337 µl (3.691 mmol) 3,4-Dihydro-2H-pyran sowie mit 3 mg (0.016 mmol) p-Toluolsulfonsäure (PTS) als Katalysator versetzt. Das Reaktionsgemisch wird 30 Minuten bei Raumtemperatur gerührt. Die Reaktionslösung wird mit Wasser verdünnt, die Phasen getrennt und die wässrige Phase dreimal mit Ethylacetat extrahiert. Anschliessend wird die organische Phase mit gesättigter Natriumhydrogencarbonat-Lösung sowie mit gesättigter Natriumchlorid-Lösung gewaschen. Die vereinigten organischen Phasen werden über Natriumsulfat getrocknet. Die Reinigung der Rohsubstanz erfolgt mit Hilfe der präparativen Säulenchromatographie (Kieselgel 60, Laufmittel: Dichlormethan/Methanol, 199:1, v/v). Hierbei können zwei Isomerengemische von 5,11-Di-O-Tetrahydropyranyl-soraphen C sowie die beiden Isomeren von 11-O-Tetrahydropyranyl-Soraphen C (108/109) isoliert werden.

H-13. Herstellung von 5-Formyl-Soraphen C (Verb. Nr. 1) und 11-Formyl-Soraphen C (Verb. Nr. 147)

40 mg (7.5 µmol) Soraphen C, 10 mg (80 µmol) 4-Dimethylaminopyridin, 3.8 µl (4.6 mg, 100 µmol) Ameisensäure und 41 µl (30 mg, 300 µmol) Triethylamin werden in 4 ml trockenem Dichlormethan gelöst. Die Lösung wird auf -15°C gekühlt, durch ein Septum mit 7.6 µl (8.2 mg, 80 µmol) Essigsäureanhydrid versetzt und 30 min bei dieser Temperatur gerührt. Dann wird das Kühlbad entfernt und weitere 30 min gerührt. Das Reaktionsgemisch wird mit einigen Tropfen Methanol versetzt und im Vakuum eingeengt. Der Rückstand wird mit 1N Chlorwasserstoffsäure versetzt und zweimal mit Ethylacetat extrahiert. Die vereinigten organischen Phasen werden mit 5%iger $NaHCO_3$-, sowie mit konz. NaCl-Lösung gewaschen und über Natriumsulfat getrocknet. Es werden 43 mg Rohprodukt erhalten, die mittels PSC gereinigt werden. Neben 11 mg Verb. Nr. 1 werden auch 4.5 mg (8 µmol, 11 %) Verb. Nr. 147 isoliert.

H-14. Herstellung von 5,11-Diformyl-Soraphen C (Verb. Nr. 148)

42 mg (83 µmol) Soraphen C, 23 mg (190 µmol) 4-Dimethylaminopyridin, 17 µl (21 mg, 450 µmol) Ameisensäure und 134 µl (97 mg, 960 µmol) Triethylamin weden in 3 ml trockenem Dichlormethan gelöst. Die Lösung wird auf -15°C gekühlt, durch ein Septum mit 36 µl (39 mg, 380 µmol) Essigsäureanhydrid versetzt und 30 min bei dieser Temperatur gerührt. Dann wird das Kühlbad entfernt und weitere 30 min gerührt. Das Reaktionsgemisch wird mit einigen Trophen Methanol versetzt und im Vakuum eingeengt. Der Rückstand wird

mit IN Chlorwasserstoffsäure versetzt und zweimal mit Ethylacetat extrahiert. Die vereinigten organischen Phasen werden mit 5%iger $NaHCO_3$-, sowie mit konz. NaCl-Lösung gewaschen und über Natriumsulfat getrocknet. Es werden 47 mg der Verb. Nr. 148 erhalten, die spektroskopisch einheitlich sind.

H-15. Herstellung von 11-Formyl-Soraphen C (Verb. Nr. 147)

27.0 mg (48 µmol) der Verb. Nr. 148 (Beispiel H-14) werden in 1 ml Methanol gelöst und mit 10 µl einer 25%igen Ammoniaklösung versetzt. Die Reaktion wird dünnschichtchromatographisch verfolgt. Nach 10 min wird die Reaktionslösung im Vakuum eingeengt, mit 1N Salzsäure versetzt und zweimal mit Ethylacetat extrahiert. Die vereinigten organischen Phasen werden mit 5%iger $NaHCO_3$-, sowie mit konz. NaCl-Lösung gewaschen und über Natriumsulfat getrocknet. Die Verbindung Nr. 147 (23 mg) wird mittels PSC gereinigt.

H-16. Herstellung von 5,11-Diacetyl-Soraphen C (Verb. Nr. 152) und 5-Acetyl-Soraphen C (Verb. Nr. 3)

50 mg (98.5 µmol) Soraphen C werden in 0.5 ml trockenem Pyridin gelöst, mit 0.3 ml (325 mg, 3.2 mmol) Essigsäureanhydrid versetzt und 1 h bei Raumtemperatur gerührt. Die Reaktionsmischung wird mit 1N Salzsäure versetzt und zweimal mit Ethylacetat extrahiert. Die vereinigten organischen Phasen werden mit 5%iger $NaHCO_3$-, sowie mit konz. NaCl-Lösung gewaschen und über Natriumsulfat getrocknet. Es werden 68 mg Rohprodukt erhalten, die mittels MPLC (Säulengrösse: 239 $cm^2$, Säulenmaterial Lichroprep Si60, 15-25 µm, Merck Art. 9336; Laufmittel: Dichlormethan/Methanol 99:1, v/v; Fluss: 6 ml/min; Druck 12 bar; $R_t$ 152=6.5 min; $R_t$ 3 = 13 min) gereinigt werden.
Ausbeute:

34 % (33 mg, 56 µmol) Verb. Nr. 152
9 % (5 mg, 9 µmol) Verb. Nr. 3.

Eine gegebenenfalls in 5-Stellung befindliche Silylgruppe (z.B. Verb. Nr. 24, 44, 69 usw.) lässt sich grundsätzlich nach Art des Herstellungsbeispiels H-3, H-5 oder H-8 unter Gewinnung der entsprechenden Soraphen C-Derivate abspalten.

H-17. Herstellung von 5-tert.Butyldimethylsilyl-11-methioninyl-Soraphen C (Verb. Nr. 211)

50,9 mg der Verbindung 2 werden zusammen mit 24,5 mg N-BOC-Methionin, 22,7 mg Dicyclohexylcarbodiimid und einer Spatelspitze 4-Dimethylaminopyridin in 2 ml Methylenchlorid gelöst und 3 Stunden bei Raumtemperatur gerührt. Die Mischung wird in Ethylacetat aufgenommen, und sukzessive mit 1N Chlorwasserstoffsäure, gesättigter Natriumhydrogencarbonat-Lösung und Sole gewaschen, über Natriumsulfat getrocknet und eingeengt. Chromatographie an Kieselgel mit Ethylacetat/Hexan (1:3) als Laufmittel liefert die am Amin geschützte Verbindung. 44 mg davon werden in 7 ml Methylenchlorid gelöst und mit 1 ml Ameisensäure versetzt. Nach 6 Stunden Rühren bei Raumtemperatur wird das Gemisch in Ethylacetat aufgenommen, mit gesättigter Natriumhydrogencarbonat-Lösung und Sole gewaschen, über Natriumsulfat getrocknet und eingeengt. Chromatographie an Kieselgel mit Methylenchlorid/Aceton (8:2) liefert 41,3 mg der Verbindung Nr. 211.

Nach Art der vorstehenden Beispiele oder nach Art einer der weiter oben angegebenen Methoden lassen sich folgende Soraphen C-Derivate der Formel I herstellen.

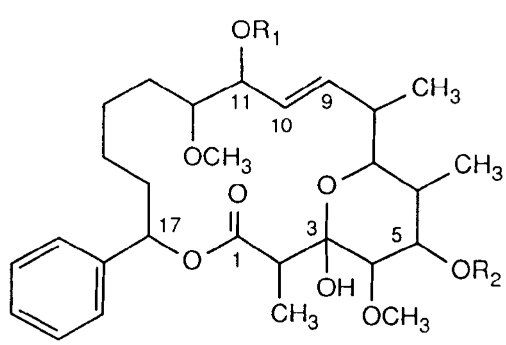

(I)

Hierin bedeuten
Si-tBu-d = tert. Butyl-dimethylsilyl

Glucosyl = 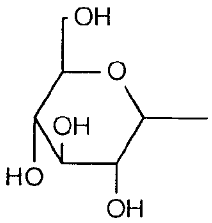 (wahlweise α- oder β-Form)

Acetylglucosyl = 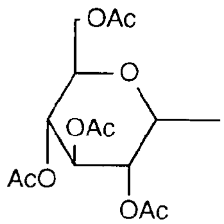 (wahlweise α- oder β-Form)

Tabelle

| Nr. | $R_1$ | $R_2$ |
|---|---|---|
| 1 | H | CHO |
| 2 | H | Si-tBu-d |
| 3 | H | $COCH_3$ |
| 4 | H | $COCH_2OCH_3$ |
| 5 | H | $Si(CH_3)_3$ |
| 6 | H | $Si(CH_3)_2$-thexyl |
| 7 | H | $Si(C_6H_5)_2$(t-Butyl) |
| 8 | H | CO-$nC_5H_{11}$ |
| 9 | H | $COCCl_3$ |
| 10 | $C_2H_5$ | H |
| 11 | $C_2H_5$ | Si-tBu-d |
| 12 | $C_2H_5$ | $COCH_3$ |
| 13 | i-$C_3H_7$ | H |
| 14 | i-$C_3H_7$ | Si-tBu-d |
| 15 | n-$C_3H_7$ | H |
| 16 | n-$C_3H_7$ | CHO |
| 17 | n-Bu | H |
| 18 | n-Bu | Si-tBu-d |
| 19 | n-Bu | $COCH_2OCH_3$ |
| 20 | t-$C_4H_9$ | H |
| 21 | t-$C_4H_9$ | Si-tBu-d |
| 22 | t-$C_4H_9$ | CO-$nC_5H_{11}$ |
| 23 | Allyl | H |
| 24 | Allyl | Si-tBu-d |
| 25 | Allyl | $Si(CH_3)_2$thexyl |
| 26 | Allyl | $COCH_2OCH_3$ |
| 27 | $-CH_2CH=CHCH_2CH_2CH_3$ | H |
| 28 | $-CH_2CH=CHCH_2CH_2CH_3$ | $Si(CH_3)_2$t-Bu |
| 29 | $-CH_2CH_2CH_2CH_2CH=CH_2$ | H |
| 30 | $-CH_2C{\equiv}CH$ | H |

| Nr. | $R_1$ | $R_2$ |
|---|---|---|
| 31 | $-CH_2C{\equiv}CH$ | Si-tBu-d |
| 32 | $-CH_2C{\equiv}CCH_2CH_3$ | H |
| 33 | $-CH_2CH_2CH_2C{\equiv}CCH_3$ | H |
| 34 | $-C_6H_5$ | H |
| 35 | $-C_6H_5$ | Si-tBu-d |
| 36 | Cyclopropyl | H |
| 37 | Cyclopropyl | Si-tBu-d |
| 38 | Cyclohexyl | H |
| 39 | Cyclopentyl | H |
| 40 | Cyclopentyl | $Si(CH_3)_3$ |
| 41 | $n-C_6H_{13}$ | H |
| 42 | $n-C_6H_{13}$ | Si-tBu-d |
| 43 | $-CH_2C_6H_5$ | H |
| 44 | $-CH_2C_6H_5$ | Si-tBu-d |
| 45 | $-CH_2C_6H_5$ | $COCF_3$ |
| 46 | $-CH_2COOH$ | H |
| 47 | $-CH_2CH_2NH_2$ | H |
| 48 | $-CH_2C_6H_4(2-NO_2)$ | H |
| 49 | $-CH_2-C_6H_4(4-OCH_3)$ | H |
| 50 | $-CH_2-C_6H_4(3-CF_3)$ | H |
| 51 | $-C_6H_4$(3-OH) | H |
| 52 | $-CH_2-C_6H_3$(2,6-dimethyl) | H |
| 53 | $-CH_2-C_6H_4$(3-Br) | H |
| 54 | $-CH_2-C_6H_4$(4F) | H |
| 55 | $CH_2CF_3$ | H |
| 56 | $CH_2CF_3$ | Si-tBu-d |
| 57 | $CH_2CH_2Br$ | H |
| 58 | $CF_3$ | H |
| 59 | $CF_3$ | Si-tBu-d |
| 60 | $CF_2CF_3$ | H |
| 61 | $CF_2CHF_2$ | H |
| 62 | $CHF_2$ | H |
| 63 | $CHF_2$ | Si-t-Bu-d |

| Nr. | $R_1$ | $R_2$ |
|---|---|---|
| 64 | $CHF_2$ | CHO |
| 65 | $CHF_2$ | $COCH_2OCH_3$ |
| 66 | $CH_2CH(OH)$Phenyl | H |
| 67 | $(CF_2)_5CF_3$ | H |
| 68 | $CH_2OCH_3$ | H |
| 69 | $CH_2OCH_3$ | Si-tBu-d |
| 70 | $CH_2OCH_3$ | CHO |
| 71 | $CH_2OCH_3$ | $COCH_3$ |
| 72 | $CH_2OCH_3$ | $Si(CH_3)_2$-thexyl |
| 73 | $CH_2SCH_3$ | H |
| 74 | $CH_2SCH_3$ | Si-tBu-d |
| 75 | $CH_2SCH_3$ | CHO |
| 76 | $CH_2SCH_3$ | $COCH_2OCH_3$ |
| 77 | $CH_2N(CH_3)_2$ | H |
| 78 | $CH_2N(CH_3)_2$ | Si-tBu-d |
| 79 | $CH_2N(CH_3)(C_6H_{13})(n)$ | H |
| 80 | $CH_2NHCH_3$ | H |
| 81 | $CH_2NHCH_3$ | Si-tBu-d |
| 82 | $CH_2OC_2H_5$ | H |
| 83 | $CH_2OC_2H_5$ | Si-tBu-d |
| 84 | $CH_2OnC_6H_{13}$ | H |
| 85 | $CH_2O$-t.-But. | H |
| 86 | $CH_2O$-t.-But. | Si-tBu-d |
| 87 | $CH_2O$-i-Prop. | H |
| 88 | $CH_2O$-i-Prop. | Si-tBu-d |
| 89 | $CH_2OCH_2$Phenyl | H |
| 90 | $CH_2OCH_2$Phenyl | Si-tBu-d |
| 91 | $CH_2OCOCH_3$ | H |
| 92 | $CH_2OCOCH_3$ | Si-tBu-d |
| 93 | $CH_2OCOCH_3$ | $COCH_3$ |
| 94 | $CH_2OCH_2CH_2OCH_3$ | H |
| 95 | $CH_2OCH_2CH_2OCH_3$ | Si-tBu-d |
| 96 | $CH_2OCH_2CH_2OCH_3$ | CHO |

| Nr. | $R_1$ | $R_2$ |
|---|---|---|
| 97 | $CH_2OCH_2CH_2OCH_3$ | $COCH_2OCH_3$ |
| 98 | $CH_2OCH_3$ | $COCH_2OCH_3$ |
| 99 | $CH_2OCH_2CH_2OCH_3$ | $COCH_3$ |
| 100 | $CH_2CH_2OCH_2CH_2OCH_3$ | H |
| 101 | $CH_2CH_2OCH_2CH_2OCH_3$ | Si-tBu-d |
| 102 | $CH_2CH_2O(CH_2CH_2O)_2CH_3$ | H |
| 103 | $CH_2CH_2O(CH_2CH_2O)_2CH_3$ | Si-tBu-d |
| 104 | $-CH(CH_3)OCH_3$ | H |
| 105 | $-CH(CH_3)OCH_3$ | Si-tBu-d |
| 106 | 2-tetrahydrofuryl | H |
| 107 | 2-tetrahydrofuryl | Si-tBu-d |
| 108 | 2-Tetrahydropyranyl Isomer A | H |
| 109 | 2-Tetrahydropyranyl Isomer B | H |
| 110 | 2-Tetrahydropyranyl | Si-tBu-d |
| 111 | 2-Tetrahydropyranyl | $COCH_3$ |
| 112 | 2-tetrahydrothienyl | H |
| 113 | 2-Tetrahydrothiopyranyl | H |
| 114 | $-CH(nC_4H_9)OCH_3$ | H |
| 115 | $-CH(CH_3)OnC_6H_{13}$ | H |
| 116 | $-C(CH_3)_2OCH_3$ | H |
| 117 | $-C(CH_3)_2OCH_3$ | Si-tBu-d |
| 118 | $-CH_2OCH(CH_2OCH_3)_2$ | H |
| 119 | $-CH_2O-C_6H_5$ | H |
| 120 | $-CH_2O-C_6H_5$ | Si-tBu-d |
| 121 | $-CH_2O-C_6H_4(4-OCH_3)$ | H |
| 122 | $-CH_2O-C_6H_4(2-NO_2)$ | H |
| 123 | $CH_2O-C_6H_4(3-F)$ | H |
| 124 | $CH_2-O-C_6H_3$(2,6-dimethyl) | H |
| 125 | $CH_2OCH_2CH_2OH$ | H |
| 126 | $CH_2OCH_2CH_2OH$ | Si-tBu-d |
| 127 | Glucosyl β-Form | H |
| 128 | Glucosyl α-Form | H |
| 129 | Glucosyl α-Form | Si-tBu-d |

| Nr. | $R_1$ | $R_2$ |
|---|---|---|
| 130 | Glucosyl β-Form | Si-tBu-d |
| 131 | Acetylglucosyl α-Form | H |
| 132 | Acetylglucosyl β-Form | H |
| 133 | Acetylglucosyl α-Form | Si-tBu-d |
| 134 | Acetylglucosyl β-Form | Si-tBu-d |
| 135 | 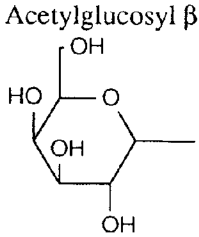 β-Form | H |
| 136 | 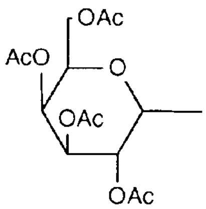 β-Form | H |
| 137<br>138 | 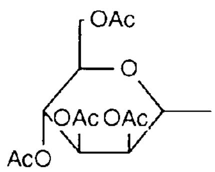 β-Form | Si-tBu-d<br>H |
| 139 | 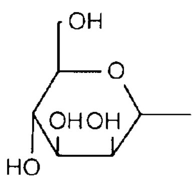 β-Form | H |
| 140 | Acetylglucosyl β-Form | $-COCH_3$ |
| 141 | 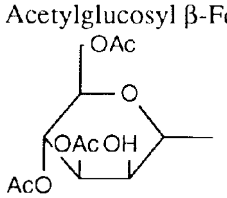 β-Form | $-COCH_3$ |

| Nr. | $R_1$ | | $R_2$ |
|---|---|---|---|
| 142 | 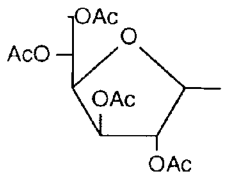 | β-Form | $-COCH_3$ |
| 143 | 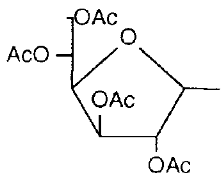 | β-Form | Si-tBu-d |
| 144 | 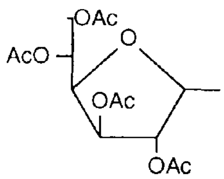 | β-Form | H |
| 145 | 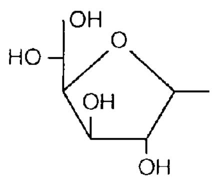 | β-Form | Si-tBu-d |
| 146 | 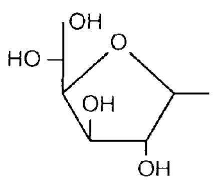 | | H |
| 147 | -CHO | | H |
| 148 | -CHO | | CHO |
| 149 | -CHO | | Si-tBu-d |
| 150 | -CHO | | $COCH_3$ |
| 151 | $-COCH_3$ | | H |
| 152 | $-COCH_3$ | | $COCH_3$ |
| 153 | $-COCH_3$ | | CHO |
| 154 | $-COCH_3$ | | Si-tBu-d |
| 155 | $-COC_2H_5$ | | H |
| 156 | -COt.-But. | | H |

| Nr. | $R_1$ | $R_2$ |
|---|---|---|
| 157 | $-COnC_6H_{13}$ | H |
| 158 | $-COCH_2OCH_3$ | H |
| 159 | $-COCH_2OCH_3$ | $COCH_2OCH_3$ |
| 160 | $-COCH_2OCH_3$ | Si-tBu-d |
| 161 | $-COCH_2OCH_3$ | $Si(Ph)_2tBut.$ |
| 162 | $-COCH_2CH_2COOH$ | H |
| 163 | $COCH_2CH_2COOH$ | $Si(CH_3)_2$-Thexyl |
| 164 | $-COC_6H_5$ | H |
| 165 | $-COC_6H_5$ | Si-tBu-d |
| 166 | $-COC_6H_5$ | $-COC_6H_5$ |
| 167 | $-COC_6H_4(4\text{-}OCH_3)$ | H |
| 168 | $-CO\text{-}C_6H_4(2\text{-}NO_2)$ | H |
| 169 | $-CO\text{-}C_6H_4(3\text{-}CF_3)$ | H |
| 170 | $-CO\text{-}C_6H_4(3\text{-}OH)$ | H |
| 171 | $-COCF_3$ | H |
| 172 | $-COCF_3$ | $COCF_3$ |
| 173 | $-COCF_3$ | Si-tBu-d |
| 174 | $-COCCl_3$ | H |
| 175 | $-COCCl_3$ | $COCCl_3$ |
| 176 | $-COCCl_3$ | Si-tBu-d |
| 177 | -COi-Prop. | H |
| 178 | $-COCH_2SCH_3$ | H |
| 179 | 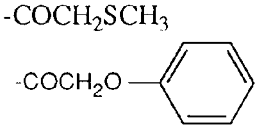 | H |
| 180 | 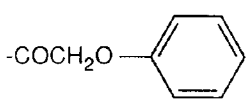 | Si-tBu-d |
| 181 | 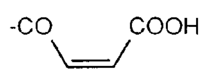 | H |
| 182 | 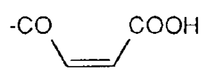 | Si-tBu-d |

| Nr. | $R_1$ | $R_2$ |
|---|---|---|
| 183 | 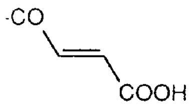 | H |
| 184 | 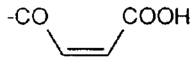 | $COCH_3$ |
| 185 | 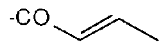 | H |
| 186 | $CO-(CH_2)_4-CH=CH_2$ | H |
| 187 | $CO-C\equiv CH$ | H |
| 188 | $CO-C\equiv CH$ | Si-tBu-d |
| 189 | $CO-(CH_2)_4-C\equiv CH$ | H |
| 190 | -CO-Cyclopropyl | H |
| 191 | -CO-Cyclopropyl | Si-tBu-d |
| 192 | -CO-Cyclohexyl | H |
| 193 | -CO-Cyclohexyl | Si-tBu-d |
| 194 | -CO-Cyclohexyl | $COCH_3$ |
| 195 | -CO-Benzyl | H |
| 196 | -CO-Benzyl | Si-tBu-d |
| 197 | $-CO-CH_2-C_6H_4(4-OCH_3)$ | H |
| 198 | $-CO-CH_2-C_6H_4(2-NO_2)$ | H |
| 199 | $-CO-CH_2-C_6H_4(4-OCH_3)$ | Si-tBu-d |
| 200 | $-CO-CH_2-C_6H_4(4-CF_3)$ | H |
| 201 | $-CO-CH_2-C_6H_3$(2,4-dimethyl) | H |
| 202 | $-COCH_2CH_2N(CH_3)_2$ | H |
| 203 | $-COCHF_2$ | H |
| 204 | $-COCF_2Cl$ | H |
| 205 | $-COCF_2Cl$ | Si-tBu-d |
| 206 | $COC_3F_7$ | H |
| 207 | $COC_3F_7$ | Si-tBu-d |
| 208 | 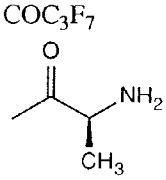 | H |

| Nr. | $R_1$ | $R_2$ |
| --- | --- | --- |
| 209 | 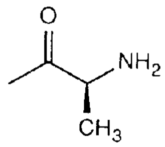 | Si-tBu-d |
| 210 | 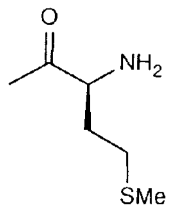 | H |
| 211 | 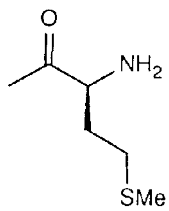 | Si-tBu-d |
| 212 | 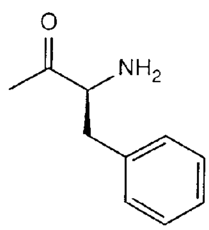 | H |
| 213 | 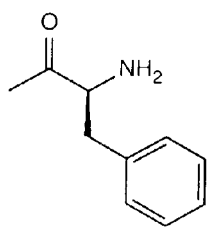 | Si-tBu-d |
| 214 | 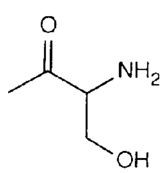 | H |

| Nr. | $R_1$ | $R_2$ |
|---|---|---|
| 215 | 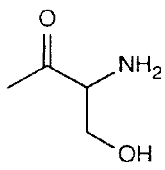 | Si-tBu-d |
| 216 | 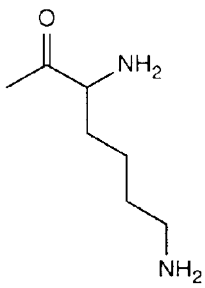 | H |
| 217 | | Si-tBu-d |
| 218 | 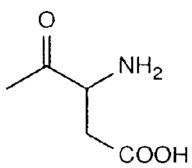 | H |
| 219 | | Si-tBu-d |
| 220 | 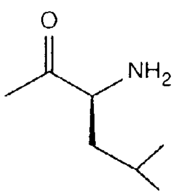 | H |
| 221 | | Si-tBu-d |
| 222 | $CH_2OC_4H_9(n)$ | Si-tBu-d |
| 223 | $CH_2OC_4H_9(n)$ | H |
| 224 | $CH(CH_3)OC_3H_7(i)$ | Si-tBu-d |
| 225 | $CH(CH_3)OC_3H_7(i)$ | H |
| 226 | $CH(CH_3)OC_3H_7(i)$ | -CHO |
| 227 | $C(CH_3)_2OC_3H_7(i)$ | Si-tBu-d |
| 228 | $C(CH_3)_2OC_3H_7(i)$ | H |
| 229 | $CH(CH_3)OC_3H_7(n)$ | Si-tBu-d |
| 230 | $CH(CH_3)OC_3H_7(n)$ | H |
| 231 | $CH(CH_3)OC_4H_9(sec)$ | Si-tBu-d |
| 232 | $CH(CH_3)OC_4H_9(sec)$ | H |
| 233 | $CH(CH_3)OC_4H_9(sec)$ | $-COCH_3$ |

| Nr. | $R_1$ | $R_2$ |
|---|---|---|
| 234 | $CH(CH_3)OC_4H_9$(tert.) | Si-tBu-d |
| 235 | $CH(CH_3)OC_4H_9$(tert.) | H |
| 236 | $CH(CH_3)OCH(CH_3)CH_2$-$OCH_3$ | Si-tBu-d |
| 237 | $CH(CH_3)OCH(CH_3)CH_2$-$OCH_3$ | H |
| 238 | $CH(CH_3)OCH(CH_3)CH_2$-$OCH_3$ | -$COCH_2OCH_3$ |
| 239 | $CH_2SC_3H_7$(i) | Si-tBu-d |
| 240 | $CH_2SC_3H_7$(i) | H |
| 241 | $CH_2SC_3H_7$(i) | -CHO |

Tabelle 2 (Physikochem. Daten einiger Soraphen C-Derivate)

? = Zuordnung unklar

| Nr. | MS $M^+$ | Lsgm.* | 2-H | $^1$H-NMR 4-H | 5-H | 11-H | Einzelnes ausgew. Proton aus $R_2$ | Rf (Lsgm)** |
|---|---|---|---|---|---|---|---|---|
| 1 | 533 ($M^+$-H) | A | 3,14 | 3,14 | 5,21 | 4,16 | 8,13 | 0,34 (1) |
| 2 | 621 ($M^+$-H) | B | 2,96 | 3,10 | 4,38 | 4,08 | 0,94 | 0,39 (2) |
| 3 | 547 ($M^+$-H) | A | 3,12 | 3,10 | 5,04 | 4,10 | 2,12 | 0,28 (1) |
| 10 | 534 | A | 3,15 | 3,19 | 4,02 | 3,81 | 1,21($CH_3$) | 0,60 (3) |
| 11 | 648 | A | 3,03 | 2,99 | 4,16 | 3,85 | 1,22 | 0,90 (2) |
| 13 | 548 | A | 3,15 | 3,19 | 4,01 | 3,85 | 1,15 | 0,36 (2) |
| 14 | 662 | A | 3,05 | 2,99 | 4,15 | 3,97 | 1,16 | 0,45 (4) |
| 17 | 562 | A | 3,12 | 3,18 | 4,02 | ? | 0,93 | 0,36 (2) |
| 18 | 676 | A | 3,04 | 2,98 | 4,16 | 3,85 | 0,93 | 0,85 (2) |
| 20 | 561 ($M^+$-H) | A | 3,14 | 3,18 | 4,04 | 3,82 | 1,22 | 0,43 (2) |
| 21 | 676 | A | 3,07 | 2,99 | 4,13 | 3,65 | 1,23 | 0,40 (4) |
| 23 | 546 | A | 3,15 | 3,19 | ? | ? | 5,96 | 0,41 (2) |
| 24 | 660 | A | 3,04 | 2,98 | 4,14 | ? | 5,98 | 0,44 (4) |
| 43 | 596 | A | 3,16 | 3,19 | 4,02 | 3,84 | 4,65 | 0,47 (2) |
| 44 | 710 | A | 3,10 | 2,98 | 4,16 | 4,01 | 4,63 | 0,54 (4) |
| 68 | 549 ($M^+$-H) | A | 3,15 | 3,19 | 4,03 | 4,14 | 4,66 AB-System | 0,36 (2) |
| 69 | - | A | 3,03 | 2,98 | 4,14 | 4,19 | 4,66 AB-System | 0,57 (2) |
| 73 | - | A | 3,15 | 3,18 | 4,03 | 4,26 | 2,21 | 0,31 (2) |
| 74 | 680 | A | 3,04 | 2,98 | 4,14 | 4,28 | 2,20 | 0,22 (4) |
| 82 | 564 | A | 3,14 | 3,18 | 4,01 | 4,15 | 4,71 | 0,24 (2) |
| 83 | - | A | 3,03 | 2,97 | 4,14 | 4,20 | 4,72 | 0,65 (2) |
| 89 | 626 | A | 3,15 | 3,18 | 4,02 | 4,22 | 4,66 AB-System | 0,31 (2) |
| 90 | - | A | 3,05 | 2,99 | 4,14 | 4,19 | 4,67 AB-System | 0,44 (4) |

| Nr. | MS $M^+$ | Lsgm.* | $^1$H-NMR 2-H | 4-H | 5-H | 11-H | Einzelnes ausgew. Proton aus $R_2$ | Rf (Lsgm)** |
|---|---|---|---|---|---|---|---|---|
| 91 | 577 ($M^-$-H) | A | 3,15 | 3,18 | 4,03 | 4,18 | 2,16 | 0,25 (2) |
| 92 | 692 | A | 3,03 | 2,97 | 4,14 | 4,22 | 2,11 | 0,23 (4) |
| 94 | 593 ($M^-$-H) | A | 3,13 | 3,18 | 4,01 | 4,18 | 4,75 AB-System | 0,13 (2) |
| 95 | 708 | A | 3,04 | 2,98 | 4,15 | 4,24 | 4,78 AB-System | 0,46 (2) |
| 101 | - | A | 3,04 | 2,99 | 4,16 | ? | 3,83 | 0,26 (4) |
| 103 | - | A | 3,04 | 2,99 | 4,16 | 3,94 | 3,82 | 0,28 (4) |
| 105 | - | A | 3,04 | 2,98 | 4,12 | 4,20 | 4,62/4,68 | 0,65 (2) |
| 108 | 590 | A | 3,12 | 3,17 | 4,00 | 4,24 | 4,54 | 0,37 (5) |
| 109 | 590 | A | 3,12 | 3,16 | 3,99 | 4,18 | 4,78 | 0,29 (5) |
| 110 | - | A | 3,08 | 2,99 | 4,15 | 4,16 4,32 | 4,67/4,79 | 0,13 (4) |
| 134 | 951 ($M^-$-H) | A | 3,02 | 2,99 | 4,15 | ? | 2,01/2,03 2,06/2,15 | 0,48 (2) |
| 147 | 534 | A | 3,12 | 3,17 | 4,01 | 5,49 | 8,11 | 0,51 (1) |
| 148 | 562 | A | 3,13 | 3,13 | 5,20 | 5,49 | 8,12 | 0,66 (1) |
| 151 | 548 | A | 3,13 | 3,16 | 4,00 | 5,36 | 2,09 | 0,41 (1) |
| 152 | 590 | A | 3,12 | 3,09 | 5,03 | 5,37 | 2,11 | 0,63 (1) |
| 158 | 578 | A | 3,13 | 3,16 | 4,00 | 5,45 | 4,06 | 0,44 (1) |
| 159 | 650 | A | 3,13 | 3,13 | 5,11 | 5,47 | 4,08 | 0,50 (1) |
| 160 | 691 ($M^-$-H) | B | 2,98 | 3,12 | 4,38 | 5,31 | 4,03 | 0,75 (2) |
| 162 | 605 ($M^-$-H) | A | 3,12 | 3,16 | 4,02 | 5,39 | 2,69 | 0,24 (6) |
| 164 | 610 | B | 3,01 | 3,10 | 4,32 | 5,14 | 8,08 | 0,56 (2) |
| 165 | 724 | B | 3,00 | 3,14 | 4,42 | 5,33 | 8,22 | 0,86 (2) |
| 181 | 627($M^-$-Na) | A | 3,02 | 3,17 | 4,23 | 5,54 | 5,83 | 0,43 (7) |
| 182 | - | C | 2,97 | 3,11 | 4,37 | 5,50 | 6,36 | 0,52 (8) |
| 210 | 638($M^+$+H) | A | 3,15 | 3,18 | 4,01 | 5,41 | 2,11 | 0,12 (9) |
| 211 | - | - | - | - | - | - - | - | 0,35 (9) |
| 212 | 654($M^+$+H) | A | 3,15 | 3,18 | 4,03 | 5,41 | 7,35 | 0,21 (9) |

| Nr. | MS $M^+$ | Lsgm.* | 2-H | $^1$H-NMR 4-H | 5-H | 11-H | Einzelnes ausgew. Proton aus $R_2$ | Rf (Lsgm)** |
|---|---|---|---|---|---|---|---|---|
| 213 | - | A | 2,98 | ? | 4,13 | 5,48 | 7,25 | 0,34 (9) |
| 220 | 620($M^+$+H) | A | 3,14 | 3,18 | 4,02 | 5,39 | 0,95 | 0,16 (9) |
| 221 | | - | - | - | - | - - | - | 0,29 (9) |
| 104 (Isomer A) | - | A | 3,14 | 3,18 | 4,02 | 4,06 | 4,72 | 0,28 (2) |
| 104 (Isomer B) | - | A | 3,14 | 3,18 | 4,02 | 4,16 | 4,65 | 0,32 (2) |
| 88 | - | A | 3,05 | 2,98 | 4,15 | 4,24 | 3,90 | 0,61 (2) |
| 87 | 577(M-H) | A | 3,14 | 3,18 | 4,03 | 4,20 | 3,94 | 0,23 (2) |
| 222 | - | A | 3,02 | 2,96 | 4,11 | 4,20 | 4,71 | 0,60 (2) |
| 223 | - | A | 3,13 | 3,17 | 4,01 | 4,18 | 4,68 | 0,29 (2) |

* Lösungsmittel für $^1$H-NMR sind:

A = $CDCl_3$

B = $CD_3COCD_3$

C = $CD_3COCD_3$-$CD_3COOD$

** Lösungsmittel für Rf-Bestimmung sind:

1 = Dichlormethan/Aceton 90:10

2 = Ethylacetat/Hexan 50:50

3 = Ethylacetat/Hexan 75:25

4 = Ethylacetat/Hexan 25:75

5 = Dichlormethan/Acetan 92:8

6 = Methanol/$H_2O$/$NH_3$ 599:400:1

7 = Methanol/$H_2O$/Essigsäure 69:30:1

8 = Chloroform/Methanol 85:15

9 = Dichlormethan/Aceton 80:20

Formulierungsbeispiele für Wirkstoffe der Formel I (% = Gewichtsprozent)

| F1. Lösungen | a) | b) | c) | d) |
|---|---|---|---|---|
| Wirkstoff aus den Tabellen | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

(MG = Molekulargewicht)

Die Lösungen sind zur Anwendung in Form kleinster Tropfen geeignet.

| F2. Granulate | a) | b) |
|---|---|---|
| Wirkstoff aus den Tabellen | 5 % | 10 % |
| Kaolin | 94 % | - |
| Hochdisperse Kieselsäure | 1 % | - |
| Attatpulgit | - | 90 % |

Der Wirkstoff wird in Methylenchlorid gelöst, auf den Träger aufgesprüht und das Lösungsmittel anschliessend im Vakuum abgedampft.

| F3. Stäubemittel | a) | b) |
|---|---|---|
| Wirkstoff aus den Tabellen | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| F4. Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F5. Emulsions-Konzentrat | |
|---|---|
| Wirdstoff aus den Tabellen | 10 % |
| Octylphenolpolyethylenglykolether | 3 % |
| (4-5 Mol Ethylenoxid) Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können mit Wasser beliebige Verdünnungen hergestellt werden.

| F6. Umhüllungs-Granulat | |
|---|---|
| Wirkstoff aus den Tabellen | 3 % |
| Polyethylenglykol (MG 200) | 3 % |
| Kaolin | 94 % |
| (MG = Molekulargewicht | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

Biologische Beispiele:

Beispiel B1: Wirkung gegen Puccinia graminis auf Weizen

a) Residual-protektive Wirkung

Weizenpflanzen werden 6 Tage nach der Aussaat mit einer aus Spritzpulver des Wirkstoffes hergestellten Spritzbrühe (60 ppm Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

b) Systemische Wirkung

Zu Weizenpflanzen wird 5 Tage nach der Aussaat eine aus Spritzpulver des Wirkstoffes hergestellte Spritzbrühe gegossen (6 ppm Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

Unbehandelte aber infizierte Kontrollpflanzen zeigten einen Puccinia-Befall von 100 %. Verbindungen aus den Tabellen zeigten gegen Puccinia-Pilze eine gute Wirkung (5-20 % Befall). Die Verbindungen Nr. 1, 3, 10, 13, 17, 20, 23, 43, 68, 73, 82, 87, 89, 91, 94, 104, 108, 109, 147, 148, 151, 158, 159, 181, 212, 213, 214, 223 und andere hemmten den Befall auf 0-5 %. Der Grundkörper "Soraphen C" hemmt im systemischen Test den Befall auf 10-20 %.

Beispiel B2: Wirkung gegen Cercospora arachidicola auf Erdnusspflanzen

a) Residual-protektive Wirkung

10-15 cm hohe Erdnusspflanzen werden mit einer aus Spritzpulver der Wirksubstanz hergestellten Spritzbrühe (20 ppm Aktivsubstanz) besprüht und 48 Stunden später mit einer Konidiensuspension des Pilzes infiziert. Die infizierten Pflanzen werden während 72 Stunden bei ca. 21°C und hoher Luftfeuchtigkeit inkubiert

und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigten Erdnusspflanzen, die mit Wirkstoffen aus den Tabellen behandelt wurden, einen stark reduzierten Cercospora-Befall. So verhinderten die Verbindungen Nr. 1, 2, 3, 10, 11, 13, 14, 17, 18, 20, 21, 23, 24, 43, 68, 73, 74, 82, 83, 87, 88, 89, 90, 91, 92, 94, 95, 101, 103, 104, 105, 108, 109, 110, 134, 147, 148, 151, 152, 158, 159, 160, 162, 164, 165, 181, 182, 210-213, 215, 220-223, 227, 228 in obigen Versuchen das Auftreten von Flecken fast vollständig (0-10 %). Die Verbindungen Nr. 68 und 94 reduzierten den Pilzbefall auch noch in einer Wirkstoffkonzentration von 2 ppm auf 0-5 %.

Beispiel B3: Wirkung gegen Erysiphae graminis auf Gerste

a) Residual-protektive Wirkung

Ca. 8 cm hohe Gerstenpflanzen werden mit einer aus Spritzpulver des Wirkstoffes hergestellten Spritzbrühe (6 ppm Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22°C aufgestellt, und der Pilzbefall wird nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (2 ppm Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Verbindungen der Formel I zeigten eine gute Wirkung gegen Erysiphae-Pilze. Unbehandelte, aber infizierte Kontrollpflanzen zeigten einen Erysiphae-Befall von 100 %. Unter anderen Verbindungen aus den Tabellen hemmten die Verbindungen Nr. 1, 2, 3, 10, 11, 13, 14, 17, 20, 23, 43, 68, 73, 82, 87, 89, 91, 94, 108, 109, 147, 151, 158, 181, 212, 223-225, 234, 236 den Pilzbefall bei Gerste auf 0 bis 5 %. Soraphen C hemmte den Pilzbefall bei diesen Konzentrationen auf 10-20 %.

Beispiel B4: Residual-protektive Wirkung gegen Venturia inaequalis auf Apfeltrieben

Apfelstecklinge mit 10-20 cm langen Frischtrieben werden mit einer aus Spritzpulver des Wirkstoffes hergestellten Spritzbrühe (20 ppm Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt. Verbindungen aus den Tabellen bewirkten eine deutliche Hemmung des Kankheitsbefalls. So hemmten die im Beispiel B-2 genannten Verbindungen den Pilzbefall fast vollständig (0-5 %). Unbehandelte aber infizierte Triebe zeigten einen 100%igen Venturia-Befall. Soraphen C hemmte den Pilzbefall auf 10-20 %.

Beispiel B5: Wirkung gegen Botrytis cinerea auf Bohnen Residual protektive Wirkung

Ca. 10 cm hohe Bohnen-Pflanzen werden mit einer aus Spritzpulver des Wirkstoffes hergestellten Spritzbrühe (60 ppm Aktivsubstanz) besprüht. Nach 48 Stunden werden die behandelten Pflanzen mit einer Konidiensuspension des Pilzes infiziert. Nach einer Inkubation der infizierten Pflanzen während 3 Tagen bei 95-100 % relativer Luftfeuchtigkeit und 21 °C erfolgt die Beurteilung des Pilzbefalls.

Der Botrytis-Befall unbehandelter aber infizierter Bohnenpflanzen betrug 100 %. Der Befall nach Behandlung mit einer der Verbindungen der Formel I betrug <20 %; bei Behandlung mit den im Beispiel B-3 genannten Verbindungen trat kein Befall auf (0-5 %). Soraphen C hemmte den Befall auf 0-5 %.

Beispiel B6: Wirkung gegen Rhizoctonia solani (Bodenpilz auf Reispflanzen)

a) Protektiv-lokale Bodenapplikation

12 Tage alte Reispflanzen werden mit einer aus einer Formulierung des Wirkstoffes hergestellten Spritzbrühe (6 ppm Aktivsubstanz), ohne oberirdische Pflanzenteile zu benetzen, angegossen. Zur Infizierung der behandelten Pflanzen wird eine Suspension von Myzel und Sklerotien von R. Solani auf die Bodenoberfläche gegeben. Nach 6-tägiger Inkubation bei 27°C (Tag), bzw. 23° (Nacht) und 100 % rel. Luftfeuchtigkeit (Feuchtkasten) in der Klimakammer wird der Pilzbefall auf Blattscheide, Blättern und Stengel beurteilt.

b) Protektiv-lokale Blattapplikation

12 Tage alte Reispflanzen werden mit einer aus einer Formulierung der Wirkstoffe hergestellten Spritzbrühe (6 ppm Aktivsubstanz) besprüht. Einen Tag später werden die behandelten Pflanzen mit einer Suspension von Myzel und Sklerotien von R. solani infiziert. Nach 6-tägiger Inkubation bei 27°C (Tag), bzw. 23°C (Nacht) und 100 %rel. Luftfeuchtigkeit (Feuchtkasten) in der Klimakammer wird der Pilzbefall auf Blattscheide, Blättern und Stengel beurteilt.

Verbindungen aus den Tabellen zeigten sehr gute Wirksamkeit durch Hemmung des Rhizoctonia-Befalls auf weniger als 10 %. Die Verbindungen Nr. 68 und 94 hemmten den Pilzbefall auf 0-5 %. Soraphen C erzielte 10-20 %. Unbehandelte jedoch infizierte Kontrollpflanzen wiesen dagegen einen Befall von 100 % auf.

Beispiel B7: Wirkung gegen Plasmopara viticola (Bert. et Curt.) (Berl. et DeToni) auf Reben

Residual-präventive Wirkung

Im Gewächshaus werden Rebenstecklinge der Sorte "Chasselas" herangezogen. Im 10-Blatt-Stadium wurden 3 Pflanzen mit einer aus der als Spritzpulver formulierten Wirksubstanz hergestellten Brühe (60 ppm Wirkstoff) besprüht. Nach dem Antrocknen des Spritzbelages werden die Pflanzen auf der Blattunterseite mit der Sporensuspension des Pilzes gleichmässig infiziert. Die Pflanzen werden anschliessend während 8 Tagen in einer Feuchtkammer gehalten. Nach dieser Zeit zeigen sich deutliche Krankheitssymptome an den Kontrollpflanzen. Anzahl und Grösse der Infektionsstellen an den behandelten Pflanzen dienen als Bewertungsmassstab für die Wirksamkeit der geprüften Substanzen.

Die Verbindungen Nr. 87, 104, 108, 109, 212, 223, 225, 226, 232, 233, 235 erzielten eine Hemmung des Krankheitsbefalls auf unter 10 %. Die Verbindungen Nr. 68 und 94 erzielten 0-5 %; Soraphen C erzielte 10-20 %.

Beispiel B8: Wirkung gegen Pyricularia oryzae auf Reispflanzen

a) Reispflanzen werden nach 2-wöchiger Anzucht mit einer aus Spritzpulver des Wirkstoffes hergestellten Spritzbrühe (60 ppm Aktivsubstanz) besprüht. Nach 48 Stunden werden die behandelten Pflanzen mit einer Konidiensuspension des Pilzes infiziert. Nach 5 Tagen Inkubation bei 95- 100 % relativer Luftfeuchtigkeit und 24°C wird der Pilzbefall beurteilt.

b) Zu 2-wöchigen Reispflanzen wird eine aus Spritzpulver des Wirkstoffes hergestellte Spritzbrühe gegossen (20 ppm Aktivsubstanz bezogen auf das Erdvolumen). Darauf werden die Töpfe mit Wasser soweit gefüllt, dass die untersten Stengelteile der Reispflanzen im Wasser stehen. Nach 96 Stunden werden die behandelten Reispflanzen mit einer Konidiensuspension des Pilzes infiziert. Nach einer Inkubation der infizierten Pflanzen während 5 Tagen bei 95 - 100 % relativer Luftfeuchtigkeit und ca. 24°C wird der Pilzbefall beurteilt.

Reispflanzen, die mit einer Spritzbrühe behandelt werden, die als Aktivsubstanz eine Verbindung aus den Tabellen 1 und 2 enthält, zeigen im Vergleich zu unbehandelten Kontrollpflanzen (100 % Befall) nur geringen Pilzbefall. So reduzierten z.B. die Verbindungen Nr. 68, 87, 104, 108, 109, 212, 223 wie Soraphen C den Befall auf 10 bis 20 %.

## Patentansprüche

1. Macrocyclische Verbindung der Formel I

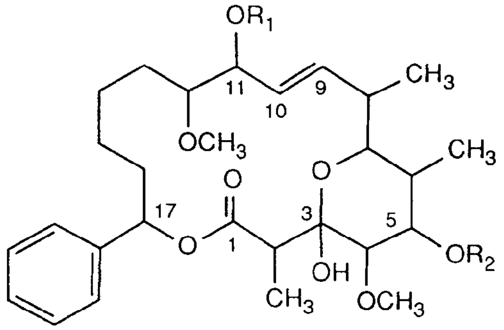

(I)

worin die Seitenkette $R_1$ maximal 15 Kohlenstoffatome besitzt und folgende Bedeutung hat:

- a) einen unsubstituierten oder substituierten Kohlenwasserstoffrest, ausgewählt aus $C_1$-$C_6$-Alkyl, $C_3$-$C_6$-Alkenyl, $C_3$-$C_6$-Alkinyl, $C_3$-$C_6$-Cycloalkyl, $C_3$-$C_6$Cycloalkyl ($C_1$-$C_6$-)alkyl, Phenyl, Xylyl und Tolyl, wobei mögliche Substituenten aus Halogen, $CF_3$, Carboxy, Phenyl, mono- und disubstituiertem Phenyl, Hydroxy, $C_1$-$C_4$Alkoxy, $C_1$-$C_4$Alkylthio, Alkoxyalkoxy, Amino, Monoalkylamino, Dialkylamino ausgewählt sind; sowie einen gesättigten unsubstituierten oder substituierten heterocyclischen Fünf- oder Sechsring, wobei dieser Ring über ein zum Heteroatom O, S oder N $\alpha$-ständigen C-Atom mit dem Sauerstoffatom in 11 -Position verbunden ist und derart ein cyclisches Acetal, Thioacetal bzw. Aminal bildet; mit der Massgabe, dass $R_1$ nicht Methyl ist;
- b) Wasserstoff, sofern der Substituent $R_2$ eine von Wasserstoff verschiedene Bedeutung hat;
- c) einen Acylrest -$COR_3$, worin $R_3$ Wasserstoff oder einen unsubstituierten oder durch Halogen, Phenyl, Phenoxy, $C_1$-$C_4$Alkoxy, $C_1$-$C_4$Alkylthio, Amino, Carboxy oder anderweitig nach Art einer Aminosäure substituierten Kohlenwasserstoffrest darstellt, der ausgewählt ist aus $C_1$-$C_6$-Alkyl, $C_2$-$C_6$-Alkenyl, $C_2$-$C_6$-Alkinyl, $C_3$-$C_6$-Cycloalkyl; oder worin $R_3$ einen unsubstituierten oder substituierten Phenylrest oder einen kernsubstituierten Benzylrest darstellt, mit einem bis drei aus Methyl, Hydroxy, Methoxy, Nitro, Halogen und/oder Trifluormethyl ausgewählten Substituenten;

während $R_2$ die Bedeutung hat:

- Wasserstoff;
- eine Silylgruppe -SiR′R″R‴, worin R′, R″ und R‴gleiche oder verschiedene Substituenten sind und $C_1$-$C_6$Alkyl oder Phenyl darstellen;
- einen Acylrest -$COR_4$, in welchem R4 Wasserstoff, Phenyl oder eine $C_1$-$C_6$Alkylgruppe darstellt, die unsubstituiert oder ein- oder mehrfach durch Halogen oder ein- bis zweifach durch $C_1$-$C_4$Alkoxy oder $C_3$-$C_6$Alkoxyalkoxy substituiert ist.

2. Eine Verbindung gemäss Anspruch 1, worin $R_1$ die genannte Bedeutung hat und $R_2$ Wasserstoff oder einen Acylrest -$COR_4$ bedeutet, in dem $R_4$ Wasserstoff oder eine $C_1$-$C_4$Alkylgruppe bedeutet, die unsubstituiert oder durch $C_1$-$C_4$Alkoxy substituiert ist.

3. Eine Verbindung gemäss Anspruch 2, worin $R_1$ zusammen mit dem Sauerstoffatom in der 11-Position ein offenes Acetal der Formel

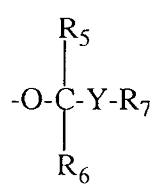

bedeutet, in dem Y Sauerstoff oder Schwefel $R_5$ und $R_6$ unabhängig Wasserstoff oder $C_1$-$C_4$Alkyl und $R_7$ einen $C_1$-$C_4$Alkylrest darstellen, der unsubstituiert ist oder, im Falle $R_7$ = $C_2$-$C_4$Alkyl, $\beta$-ständig bis endständig mit 1-3 Substituenten, ausgewählt aus OH, Alkoxyalkoxy mit maximal 6 C-Atomen und $C_1$-$C_4$-Alkoxy, substituiert sein kann;

oder worin $R_1$ ein ringförmiges Acetal der Formel

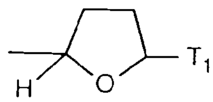 oder 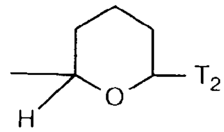

darstellt, das nach Art einer Furanose oder Pyranose ganz oder teilweise durch -OH oder -O-COCH$_3$ substituiert ist, und worin T$_1$ Wasserstoff, OH, -CH$_2$OH, -CHOH-CH$_2$OH oder ihre Acetylderivate und T$_2$ Wasserstoff, OH, CH$_2$OH oder deren Acetylderivate bedeuten;
oder worin R$_1$ ein Thioacetal der Formel

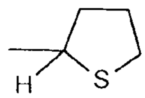 oder 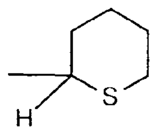

darstellt.

4. Eine Verbindung gemäss Anspruch 3, worin R$_2$ Wasserstoff bedeutet.

5. Eine Verbindung gemäss Anspruch 3, worin R$_1$ zusammen mit dem Sauerstoffatom in der 11-Position ein offenes Acetal der Formel

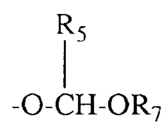

bedeutet, in dem R$_5$ Wasserstoff oder Methyl ist und R$_7$ einen C$_1$-C$_4$-Alkylrest darstellt, der unsubstituiert ist oder im Falle von R$_7$= C$_2$-C$_4$Alkyl β-ständig bis endständig durch ein bis drei C$_1$-C$_3$Alkoxygruppen substituiert sein kann.

6. Eine Verbindung gemäss Anspruch 5, worin R$_2$ Wasserstoff, Formyl, Acetyl oder Methoxyacetyl bedeutet.

7. Eine Verbindung gemäss Anspruch 6, worin R$_1$ die Gruppe CH$_2$-O-CH$_3$ und R$_2$ Wasserstoff bedeuten. (= Verb. 68)

8. Eine Verbindung gemäss Anspruch 6, worin R$_1$ die Gruppe CH$_2$-O-CH$_2$CH$_2$OCH$_3$ und R$_2$ Wasserstoff bedeuten. (= Verb. 94)

9. Eine Verbindung gemäss Anspruch 2, worin R$_1$ einen C$_3$-C$_5$Cycloalkylrest oder einen unsubstituierten oder β-ständig bis endständig substituierten Kohlenwasserstoffrest bedeutet, ausgewählt aus C$_2$-C$_3$Alkyl, C$_3$-C$_4$Alkenyl und C$_3$-C$_4$Alkinyl, und dessen Substituenten ausgewählt sind aus OH, C$_1$-C$_4$Alkoxy und Alkoxyalkoxy mit maximal 6 Kohlenstoffatomen.

10. Eine Verbindung gemäss Anspruch 9, worin R$_2$ Wasserstoff, Formyl, Acetyl oder Methoxyacetyl bedeutet.

11. Eine Verbindung gemäss Anspruch 1, worin R$_1$ den Carbonsäurerest -COR$_3$ einer Aminosäure darstellt.

12. Eine Verbindung gemäss Anspruch 11, worin R$_2$ Wasserstoff, Formyl, Acetyl oder Methoxyacetyl bedeutet.

13. Eine Verbindung gemäss Anspruch 1, worin R$_1$ Wasserstoff ist und R$_2$ eine Silylgruppe -SiR′R″R‴ bedeutet, worin R′, R″ und R‴ gleiche oder verschiedene Substituenten, ausgewählt aus C$_1$-C$_6$-Alkyl und Phenyl, darstellen.

14. Verfahren zur Herstellung einer macrocyclischen Verbindung der Formel I nach Anspruch 1, dadurch gekennzeichnet, dass man Soraphen C in geeigneter Auswahl und Reihenfolge einer Verätherung,

Silylierung, (Thio)Acetalisierung, Aminalisierung, Acylierung, Silyletherspaltung und/oder Esterspaltung mit dem jeweils gewünschten Reaktionspartner unterwirft.

**15.** Verfahren zur Bekämpfung und Verhütung eines Befalls von Pflanzen durch Mikroorganismen, dadurch gekennzeichnet, dass man eine macrocyclische Verbindung der Formel I auf die Pflanze, auf Pflanzenteile oder auf den Nährboden der Pflanze appliziert.

**16.** Mittel zur Bekämpfung und Verhütung eines Befalls von Pflanzen durch Mikroorganismen, dadurch gekennzeichnet, dass es als Wirkstoff eine Verbindung der Formel I zusammen mit einem geeigneten Trägermaterial enthält.

**17.** Mittel nach Anspruch 16, dadurch gekennzeichnet, dass es als Wirkstoff eine Verbindung gemäss einem der Ansprüche 2-13 enthält.

Europäisches Patentamt

# EUROPÄISCHER RECHERCHENBERICHT

Nummer der Anmeldung
EP 92 81 0815

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y,D | EP-A-0 358 606 (GESELLSCHAFT FÜR BIOTECHNOLOGISCHE FORSCHUNG)<br>* Ansprüche 1,9-11 * | 1-17 | C07H19/01<br>A01N43/90 |
| Y | EP-A-0 358 608 (GESELLSCHAFT FÜR BIOTECHNOLOGISCHE FORSCHUNG)<br>* Ansprüche 1,10-15 * | 1-17 | |
| Y | EP-A-0 282 455 (GESELLSCHAFT FÜR BIOTECHNOLOGISCHE FORSCHUNG)<br>* Ansprüche 1,12 * | 1-17 | |
| | | | RECHERCHIERTE SACHGEBIETE (Int. Cl.5 ) |
| | | | C07H<br>A01N |

Der vorliegende Recherchenbericht wurde für alle Patentansprüche erstellt

| Recherchenort | Abschlußdatum der Recherche | Prüfer |
|---|---|---|
| DEN HAAG | 12 FEBRUAR 1993 | BRENNAN J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)